# EUROPEAN PATENT APPLICATION

(11) **EP 3 590 506 A1**
(43) Date of publication of application: **08.01.2020**
(21) Application number: 19175798.8
(22) Date of filing: 20.05.2016
(51) Int. Cl.: A61K 9/20, A61K 31/4015, A61K 9/00, A61P 25/00, A61P 25/28, A61P 25/08

(54) **EXTENDED RELEASE PHARMACEUTICAL COMPOSITIONS OF LEVETIRACETAM**

(30) Priority: 22.05.2015 US 201562165812 P
(62) Divisional of application: 16728462.9
(71) Applicant: Agenebio, Inc., Baltimore, Maryland 21218 (US)
(72) Inventor: GALLAGHER, Michela, Baltimore, MD 21230 (US); ROSENZWEIG-LIPSON, Sharon, East Brunswick, NJ 08816 (US); MELSOPP, Elsie, Wilmington, NC 28411 (US); JAMES, Jack, Lawrence, Castle Hayne, NC 28429 (US)
(74) Representative: Garner, Stephen

(57) **Abstract**

This invention relates to novel extended release pharmaceutical compositions of levetiracetam and preparations and characterizations thereof. This invention further relates to using these extended release pharmaceutical compositions of levetiracetam for the treatment of cognitive impairment associated with central nervous system (CNS) disorders in a subject in need or at risk thereof.

## Description

This application claims priority and benefit from U.S. Provisional Patent Application 62/165,812, filed May 22, 2015, and U.S. Non-Provisional Patent Application 15/160,424, filed May 20, 2016, the contents and disclosures of which are incorporated herein by reference in their entirety.

### Field of the Invention

This invention relates to novel extended release pharmaceutical compositions of levetiracetam and preparations and characterizations thereof. This invention further relates to using these extended release pharmaceutical compositions of levetiracetam for the treatment of cognitive impairment associated with central nervous system (CNS) disorders in a subject in need or at risk thereof.

### Background of the Invention

Cognitive ability may decline as a normal consequence of aging or as a consequence of a CNS disorder.

For example, a significant population of elderly adults experiences a decline in cognitive ability that exceeds what is typical in normal aging. Such age-related loss of cognitive function is characterized clinically by progressive loss of memory, cognition, reasoning, and judgment. Mild Cognitive Impairment (MCI), Age-Associated Memory Impairment (AAMI), Age-Related Cognitive Decline (ARCD) or similar clinical groupings are among those related to such age-related loss of cognitive function. According to some estimates, there are more than 16 million people with AAMI in the U.S. alone (Barker et al., 1995), and MCI is estimated to affect 5.5 - 7 million in the U.S. over the age of 65 (Plassman et al., 2008).

Cognitive impairment is also associated with other central nervous system (CNS) disorders, such as dementia, Alzheimer's Disease(AD), prodromal AD, post traumatic stress disorder (PTSD), schizophrenia, bipolar disorder (e.g., mania), amyotrophic lateral sclerosis (ALS), cancer-therapy-related cognitive impairment, mental retardation, Parkinson's disease (PD), autism, compulsive behavior, and substance addiction.

There is, therefore, a need for effective treatment of cognitive impairment associated with central nervous system (CNS) disorders and to improve cognitive function in patients diagnosed with, for example, age-related cognitive impairment, MCI, amnestic MCI, AAMI, ARCD, dementia, Alzheimer's Disease (AD), prodromal AD, post traumatic stress disorder (PTSD), schizophrenia, bipolar disorder (e.g., mania), amyotrophic lateral sclerosis, cancer-therapy-related cognitive impairment, mental retardation, Parkinson's disease (PD), autism, compulsive behavior, and substance addiction, and similar central nervous system (CNS) disorders associated with cognitive impairment or at risk of developing them.

Levetiracetam is a widely used antiepileptic drug. Its International Union of Pure and Applied Chemistry (IUPAC) name is (2S)-2-(2-oxopyrrolidin-1-yl) butanamide) and its chemical structure is shown in Formula I.

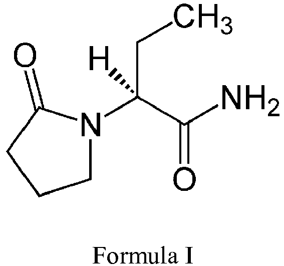

Levetiracetam is indicated as adjunctive therapy in the treatment of partial onset seizures, or myoclonic seizures, or primary generalized tonic-clonic seizures. It is recommended that such treatments should be initiated with a daily dose of 1000 mg/day. Additional dosing increments may be given to a maximum recommended daily dose of 3000 mg. Levetiracetam is currently available as immediate and extended release formulations for oral administration. Extended release dosage form of levetiracetam is available in strengths of 500 mg, 750 mg, and 1000 mg for once daily usage. Immediate release dosage form of levetiracetam is available in strengths of 250 mg, 500 mg, 750 mg, and 1000 mg for twice daily usage.

International Application Nos. PCT/US09/05647, PCT/US12/24556, and PCT/US14/29170 disclose that levetiracetam, when administered at a dose lower than the therapeutic doses for treating epilepsy, can treat cognitive impairment associated with central nervous system (CNS) disorders in a subject in need or at risk thereof.

The currently commercially available extended release dosage forms of levetiracetam comprise 500 mg, 750 mg, and 1000 mg of levetiracetam. Such extended release dosage forms are not suitable for treating cognitive impairment. There is, therefore, a need for novel extended release compositions of levetiracetam for treating cognitive impairment.

### Summary of the Invention

In one aspect, the present invention provides an extended release pharmaceutical composition comprising: a) 220 mg of levetiracetam; b) 280 mg-350 mg of hydroxypropyl methylcellulose; c) 1.2 mg-1.4 mg of colloidal silicon dioxide; d) 92.8 mg-119.2 mg of silicified microcrystalline cellulose; and e) 6.0 mg-6.7 mg of magnesium stearate. In another aspect, the present invention provides an extended release pharmaceutical composition comprising: a) 220 mg of levetiracetam; b) 280 mg of hydroxypropyl methylcellulose; c) 1.2 mg of colloidal silicon dioxide; d) 92.8 mg of silicified microcrystalline cellulose; and e) 6.0 mg of magnesium stearate. In another aspect, the present invention provides an extended release pharmaceutical composition comprising: a) 220 mg of levetiracetam; b) 347.5 mg of hydroxypropyl methylcellulose; c) 1.4 mg of colloidal silicon dioxide; d) 119.2 mg of silicified microcrystalline cellulose; and e) 6.7 mg of magnesium stearate. In certain embodiments of these aspects of the invention, the hydroxypropyl methylcellulose is Methocel™ K15M CR or Methocel™ K100M Premium CR. In certain embodiments of these aspects of the invention, the hydroxypropyl methylcellulose is Methocel™ K15M CR. In certain embodiments of these aspects of the invention, the silicified microcrystalline cellulose is ProSolv™ HD90.

In another aspect, the present invention provides an extended release pharmaceutical composition comprising: a) 190 mg of levetiracetam; b) 300 mg of hydroxypropyl methylcellulose; c) 1.2 mg of colloidal silicon dioxide; d) 102.8 mg of silicified microcrystalline cellulose; and e) 6 mg of magnesium stearate. In another aspect, the present invention provides an extended release pharmaceutical composition comprising: a) 190 mg of levetiracetam; b) 300 mg of hydroxypropyl methylcellulose; c) 1.2 mg of colloidal silicon dioxide; d) 102.8 mg of anhydrous dicalcium phosphate; and e) 6 mg of magnesium stearate. In certain embodiments of these aspects of the invention, the hydroxypropyl methylcellulose is Methocel™ K15M CR or Methocel™ K100M Premium CR. In certain embodiments of these aspects of the invention, the hydroxypropyl methylcellulose is Methocel™ K15M CR. In certain embodiments of these aspects of the invention, the silicified microcrystalline cellulose is ProSolv™ HD90.

In certain embodiments of the invention, the extended release pharmaceutical composition of the invention is formulated for once daily administration.

In certain embodiments of the invention, the extended release pharmaceutical composition of the invention is formulated for one-unit-dosage-form-once-daily administration.

In certain embodiments of the invention, the extended release pharmaceutical composition of the invention is in the form of a tablet. In some embodiments, the extended release pharmaceutical composition of the invention is in a tablet form and is formulated for one-tablet-once-daily administration.

In certain embodiments of the invention, the extended release pharmaceutical composition of the invention is formulated for oral administration.

In certain embodiments of the invention, the extended release pharmaceutical composition of the invention does not comprise a hydrophobic rate controlling polymer.

In certain embodiments of the invention, the extended release pharmaceutical composition of the invention does not comprise a functional coating.

In another aspect, this invention provides methods of improving cognition in a subject suffering from cognitive impairment or at risk thereof by administering the extended release pharmaceutical compositions of the invention. In certain embodiments, the subject suffers from cognitive impairment associated with a central nervous system (CNS) disorder, or at risk thereof. In certain embodiments, the cognitive impairment is associated with age-related cognitive impairment. In certain embodiments, the age-related cognitive impairment is Mild Cognitive Impairment. In certain embodiments, the Mild Cognitive Impairment is amnestic Mild Cognitive Impairment. In certain embodiments, the cognitive impairment is associated with dementia, Alzheimer's disease, schizophrenia, amyotrophic lateral sclerosis, post traumatic stress disorder, cancer therapy, bipolar disorder mental retardation, Parkinson's disease, autism, compulsive behavior, or substance addiction.

In another aspect, this invention provides methods of treating mild cognitive impairment due to Alzheimer's disease in a human subject in need thereof by administering the extended release pharmaceutical compositions of the invention.

In another aspect, this invention provides methods of treating amnestic mild cognitive impairment due to Alzheimer's disease in a human subject in need thereof by administering the extended release pharmaceutical compositions of the invention.

In another aspect, this invention provides methods of slowing the progression of mild cognitive impairment due to Alzheimer's disease in a human subject in need thereof by administering the extended release pharmaceutical compositions of the invention.

In another aspect, this invention provides methods of slowing the progression of amnestic mild cognitive impairment due to Alzheimer's disease in a human subject in need thereof by administering the extended release pharmaceutical compositions of the invention.

### Brief Description of the Drawings

**Figure 1** is a flow diagram of one embodiment of a process for manufacturing extended release compositions of levetiracetam (e.g., 190 mg and 220 mg tablets listed in **Tables 1 and 3**).

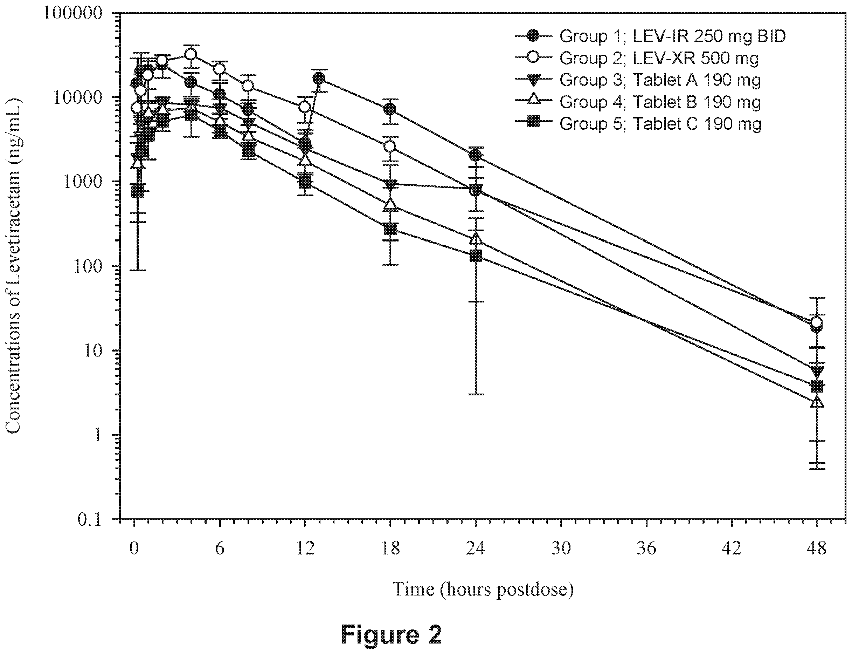

**Figure 2** shows the mean concentrations of different levetiracetam formulations in plasma following oral administration to male dogs. The tested levetiracetam formulations are: an immediate release 250 mg levetiracetam (LEV-IR) tablet being administered as 250 mg oral BID (twice daily) regimen (total dose of 500 mg); an extended release 500 mg levetiracetam tablet (LEV-XR) being administered as a single oral dose of 500 mg; the 190 mg Tablets A, B, and C of **Table 1** being administered as single oral doses of 190 mg. Plasma pharmacokinetic samples are collected at pre-dose (*i.e.,* 0), 0.25, 0.5, 1, 2, 4, 6, 8, 12, 13 (LEV-IR 250 mg BID only), 18, 24, and 48 hours post dose. For LEV-IR 250 mg BID, the 12-hour blood sample is collected just prior to administration of the second dose.
**Figure 3** shows the mean concentrations of different levetiracetam formulations in plasma following oral administration to male dogs. The tested levetiracetam formulations are: an extended release 500 mg levetiracetam tablet (LEV-XR) being administered as a single oral dose of 500 mg; the 220 mg Tablets D and E of **Table 3** being administered as single oral doses of 220 mg.
**Figure 4** shows the mean levetiracetam concentration-time profiles after administration of the 190 mg Tablet A of **Table 1** under Fasted Conditions (Group 1/Treatment A: A1) and the 190 mg Tablet A of **Table 1** under Fed Conditions (Group 1/Treatment B: B1).
**Figure 5** shows the mean levetiracetam concentration-time profiles after administration of the 220 mg Tablet D of **Table 3** under Fasted Conditions (Group 2/Treatment A: A2) and the 220 mg Tablet D of **Table 3** under Fed Conditions (Group 2/Treatment B: B2).
**Figure 6** shows the effective plasma level ranges based on Aged-Impaired rat studies and phase II study in aMCI patients. The acceptable range goal for the Phase I food effect study of the extended release formulations is established based on the effective plasma level range in aged-impaired rats and in aMCI patients, *i.e.,* between 1.9 and 4.4 µg/ml. The preferred range goal for the Phase I food effect study of the extended release formulations is established based on the effective plasma level range in aMCI patients, *i.e.,* between 2.9 and 4.4 µg/ml.

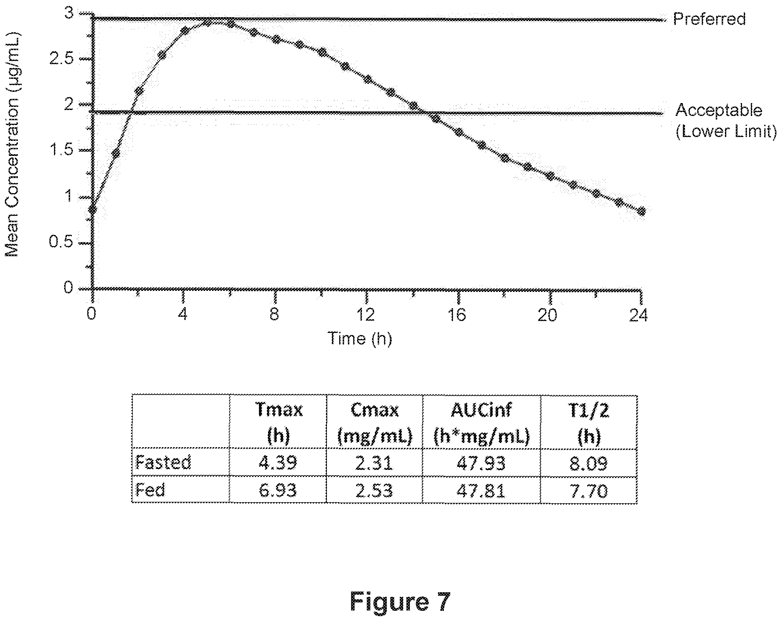

**Figure 7** shows the steady state modeling of the PK profile of the 190 mg Tablet A of **Table 1,** indicating that this tablet meets the acceptable range goal, *i.e.,* between 1.9 and 4.4 µg/ml.

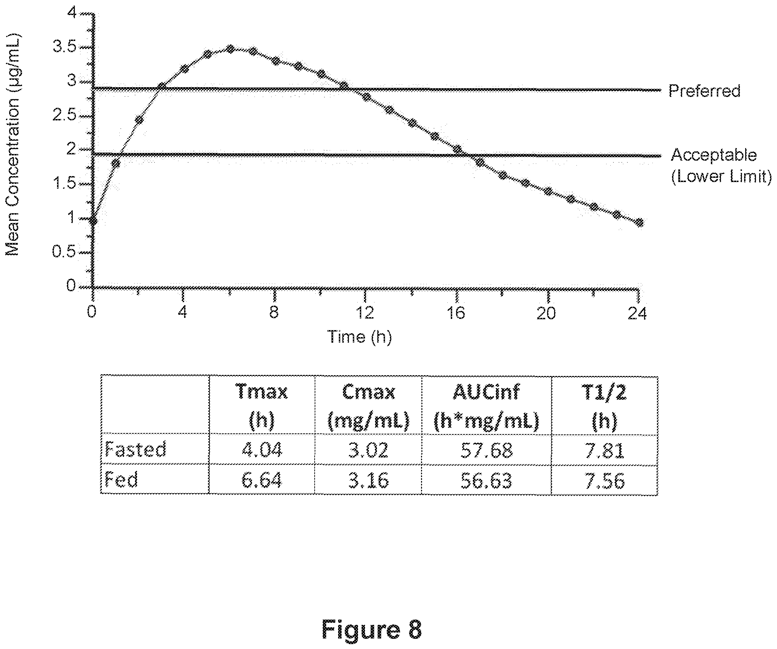

**Figure 8** shows the steady state modeling of the PK profile of the 220 mg Tablet D of Table 3, indicating that this tablet meets the preferred range goal, *i.e.,* between 2.9 and 4.4 µg/ml.
**Figure 9** is a flow diagram of another embodiment of a process for manufacturing extended release compositions of levetiracetam (*e.g*., 190 mg and 220 mg tablets listed in **Tables 1 and 3**).

### Detailed Description of the Invention

This invention provides novel extended release pharmaceutical compositions of levetiracetam. This invention also provides methods of using these extended release pharmaceutical compositions of levetiracetam for treating cognitive impairment or improving cognitive function associated with central nervous system (CNS) disorders in a subject in need or at risk thereof. This invention also provides using these extended release pharmaceutical compositions of levetiracetam in the manufacture of medicaments for treating cognitive impairment or improving cognitive function associated with central nervous system (CNS) disorders in a subject in need or at risk thereof.

In order that the invention herein described may be fully understood, the following details description is set forth.

Unless otherwise defined herein, scientific and technical terms used in this application shall have the meanings that are commonly understood by those of ordinary skill in the art to which this invention belongs. Generally, nomenclature used in connection with, and techniques of, cell and tissue culture, molecular biology, cell biology, cancer biology, neurobiology, neurochemistry, virology, immunology, microbiology, genetics, protein and nucleic acid chemistry, chemistry, and pharmacology described herein, are those well known and commonly used in the art. Each embodiment of the inventions described herein may be taken alone or in combination with one or more other embodiments of the inventions.

The methods and techniques of the present invention are generally performed, unless otherwise indicated, according to conventional methods well known in the art and as described in various general and more specific references that are cited and discussed throughout this specification. See, e.g. "Principles of Neural Science", McGraw-Hill Medical, New York, N.Y. (2000); Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); Gilbert et al., "Developmental Biology, 6th ed.", Sinauer Associates, Inc., Sunderland, MA (2000).

Chemistry terms used herein are used according to conventional usage in the art, as exemplified by "The McGraw-Hill Dictionary of Chemical Terms", Parker S., Ed., McGraw-Hill, San Francisco, C.A. (1985).

All of the above, and any other publications, patents and published patent applications referred to in this application are specifically incorporated by reference herein. In case of conflict, the present specification, including its specific definitions, will control.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

In order to further define the invention, the following terms and definitions are provided herein.

### Definitions

The term "extended release", ""extended release form", or "extended release dosage form" is widely recognized in the art of pharmaceutical sciences as systems that maintains therapeutic blood or plasma or tissue levels of a drug for an extended period. An extended release dosage form potentially provides greater effectiveness in the treatment of chronic diseases or conditions; greater convenience; reduces side effects and provides higher levels of patient compliance or therapeutic performance due to a simplified dosage schedule, compared with those of immediate-release drugs. Extended release pharmaceutical products are formulated to release the active ingredient gradually and predictably over an extended time period, such as a 24-hour period.

The term "extended release", "extended release form", or "extended release dosage form" is used herein to refer to a controlled release of levetiracetam from a dosage form to an environment over (throughout or during) an extended period of time, e.g., twenty-four hours. An extended release dosage form will release drug at substantially constant rate over an extended period of time or a substantially constant amount of drug will be released incrementally over an extended period of time. The term "extended release" used herein includes the terms "controlled release", "prolonged release", "sustained release", "slow release", or "modified release" as these terms are used in the pharmaceutical sciences.

The term "active ingredient" "active pharmaceutical ingredient" or "API" as used herein is defined as a substance which has a therapeutic effect, such as levetiracetam.

A "patient", "subject", or "individual" are used interchangeably and refer to either a human or a non-human animal. These terms include mammals, such as humans, primates, livestock animals (including bovines, porcines, etc.), companion animals (*e.g.,* canines, felines, etc.) and rodents (*e.g.,* mice and rats).

"Cognitive function" or "cognitive status" refers to any higher order intellectual brain process or brain state, respectively, involved in learning and/or memory including, but not limited to, attention, information acquisition, information processing, working memory, short-term memory, long-term memory, anterograde memory, retrograde memory, memory retrieval, discrimination learning, decision-making, inhibitory response control, attentional set-shifting, delayed reinforcement learning, reversal learning, the temporal integration of voluntary behavior, and expressing an interest in one's surroundings and self-care, speed of processing, reasoning and problem solving and social cognition.

In humans, cognitive function may be measured, for example and without limitation, by measuring neuronal injury, measuring change in Entorhinal Cortex thickness using structural MRI (*e.g.,* for measuring neuronal injury); the clinical global impression of change scale (CIBIC-plus scale); the Mini Mental State Exam (MMSE); the Neuropsychiatric Inventory (NPI); the Clinical Dementia Rating Scale (CDR) (global, memory box); the Cambridge Neuropsychological Test Automated Battery (CANTAB); the Sandoz Clinical Assessment-Geriatric (SCAG); the Buschke Selective Reminding Test (Buschke and Fuld, 1974); the Verbal Paired Associates subtest; the Logical Memory subtest; the Visual Reproduction subtest of the Wechsler Memory Scale-Revised (WMS-R) (Wechsler, 1997); the Benton Visual Retention Test; or the explicit 3-alternative forced choice task; or MATRICS consensus neuropsychological test battery; or ADAS-Cog 13 item-scale; Wechsler Logical Memory I and II; BSP-O; Neuropsychological tests (Trails A and B, BNT, SR, CFT, R-O, Paired-associates), other MRI measures, DTI, resting fMRI, and the GDS. *See* Folstein et al., J Psychiatric Res 12: 189-98, (1975); Robbins et al., Dementia 5: 266-81, (1994); Rey, L'examen clinique en psychologie, (1964); Kluger et al., J Geriatr Psychiatry Neurol 12:168-79, (1999); Marquis et al., 2002 and Masur et al., 1994. Also see Buchanan, R.W., Keefe, R.S.E., Umbricht, D., Green, M.F., Laughren, T., and Marder, S.R. (2011), The FDA-NIMH-MATRICS guidelines for clinical trial design of cognitive-enhancing drugs: what do we know 5 years later? Schizophr. Bull. 37, 1209-1217.

In animal model systems, cognitive function may be measured in various conventional ways known in the art, including using a Morris Water Maze (MWM), Barnes circular maze, elevated radial arm maze, T maze or any other mazes in which the animals use spatial information. Cognitive function can be assessed by reversal learning, extradimensional set shifting, conditional discrimination learning and assessments of reward expectancy. Other tests known in the art may also be used to assess cognitive function, such as novel object recognition and odor recognition tasks.

Cognitive function may also be measured using imaging techniques such as Positron Emission Tomography (PET), functional magnetic resonance imaging (fMRI), Single Photon Emission Computed Tomography (SPECT), or any other imaging technique that allows one to measure brain function. In animals, cognitive function may also be measured with electrophysiological techniques.

"Promoting" cognitive function refers to affecting impaired cognitive function so that it more closely resembles the function of a normal, unimpaired subject. Cognitive function may be promoted to any detectable degree, but in humans preferably is promoted sufficiently to allow an impaired subject to carry out daily activities of normal life a level of proficiency as close as possible to a normal, unimpaired subject or an age-matched normal, unimpaired subject.

In some cases, "promoting" cognitive function in a subject affected by age-related cognitive refers to affecting impaired cognitive function so that it more closely resembles the function of an aged-matched normal, unimpaired subject, or the function of a young adult subject. Cognitive function of that subject may be promoted to any detectable degree, but in humans preferably is promoted sufficiently to allow an impaired subject to carry out daily activities of normal life at a level of proficiency as close as possible to a normal, unimpaired subject or a young adult subject or an age-matched normal, unimpaired subject.

"Preserving" cognitive function refers to affecting normal or impaired cognitive function such that it does not decline or does not fall below that observed in the subject upon first presentation or diagnosis, or delays such decline.

"Improving" cognitive function includes promoting cognitive function and/or preserving cognitive function in a subject.

"Cognitive impairment" refers to cognitive function in subjects that is not as robust as that expected in a normal, unimpaired subject. In some cases, cognitive function is reduced by about 5%, about 10%, about 30%, or more, compared to cognitive function expected in a normal, unimpaired subject. In some cases, "cognitive impairment" in subjects affected by aged-related cognitive impairment refers to cognitive function in subjects that is not as robust as that expected in an aged-matched normal, unimpaired subject, or the function of a young adult subject (*i.e.* subjects with mean scores for a given age in a cognitive test).

"Treating" a condition or patient refers to taking steps to obtain beneficial or desired results, including clinical results. Beneficial or desired clinical results include, but are not limited to, improving cognitive function, delaying or slowing the progression of cognitive impairment, reducing the rate of decline of cognitive function, preventing or slowing the progression of the disease or disorder, or alleviation, amelioration, or slowing the progression, of one or more symptoms associated of cognitive impairment associated with CNS disorders, such as age-related cognitive impairment, Mild Cognitive Impairment (MCI), amnestic MCI, dementia, Alzheimer's Disease (AD), prodromal AD, PTSD, schizophrenia or bipolar disorder (in particular, mania), amyotrophic lateral sclerosis (ALS) or cancer therapy-related cognitive impairment. Treating age-related cognitive impairment further comprises slowing the conversion of age-related cognitive impairment (including, but not limited to MCI, ARCD and AAMI) into dementia (*e.g.*, AD).

"Treating cognitive impairment" refers to taking steps to improve cognitive function in a subject with cognitive impairment so that the subject's performance in one or more cognitive tests is improved to any detectable degree, or is prevented from further decline. Preferably, that subject's cognitive function, after treatment of cognitive impairment, more closely resembles the function of a normal, unimpaired subject. Treatment of cognitive impairment in humans may improve cognitive function to any detectable degree, but is preferably improved sufficiently to allow the impaired subject to carry out daily activities of normal life at the same level of proficiency as a normal, unimpaired subject. In some cases, "treating cognitive impairment" refers to taking steps to improve cognitive function in a subject with cognitive impairment so that the subject's performance in one or more cognitive tests is improved to any detectable degree, or is prevented from further decline. Preferably, that subject's cognitive function, after treatment of cognitive impairment, more closely resembles the function of a normal, unimpaired subject. In some cases, "treating cognitive impairment" in a subject affecting by age-related cognitive impairment refers to takings steps to improve cognitive function in the subject so that the subject's cognitive function, after treatment of cognitive impairment, more closely resembles the function of an age-matched normal, unimpaired subject, or the function of a young adult subject. In some cases, "treating cognitive impairment" in a subject refers to taking steps to delay or slow the progression of cognitive impairment in a subject with cognitive impairment. In some cases, "treating cognitive impairment" in a subject refers to taking steps to reduce the rate of decline of cognitive function in a subject with cognitive impairment.

The term "agent" is used herein to denote a chemical compound (such as an organic or inorganic compound, a mixture of chemical compounds), a biological macromolecule (such as a nucleic acid, an antibody, including parts thereof as well as humanized, chimeric and human antibodies and monoclonal antibodies, a protein or portion thereof, *e.g.,* a peptide, a lipid, a carbohydrate), or an extract made from biological materials such as bacteria, plants, fungi, or animal (particularly mammalian) cells or tissues. Agents include, for example, agents which are known with respect to structure, and those which are not known with respect to structure.

### Description of Compositions of the Invention

This invention provides extended release compositions of levetiracetam. The compositions of this invention can be used for improving cognition in patients who suffer from cognitive impairment associated with central nervous system (CNS) disorders in a subject in need or at risk thereof. The compositions of this invention is administered once a day (*i.e.*, once every 24 hours) for improving cognition.

In one aspect, the invention provides extended release pharmaceutical compositions comprising: a) 220 mg of levetiracetam; b) 280 mg to 350 mg of hydroxypropyl methylcellulose (or hypromellose); c) 1.2 mg to 1.4 mg of colloidal silicon dioxide; d) 92.8 mg-119.2 mg of silicified microcrystalline cellulose; and e) 6.0 mg to 6.7 mg of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 220 mg of levetiracetam; b) 280 mg of hydroxypropyl methylcellulose (or hypromellose); c) 1.2 mg of colloidal silicon dioxide; d) 92.8 mg of silicified microcrystalline cellulose; and e) 6.0 mg of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 220 mg of levetiracetam; b) 347.5 mg of hydroxypropyl methylcellulose (or hypromellose); c) 1.4 mg of colloidal silicon dioxide; d) 119.2 mg of silicified microcrystalline cellulose; and e) 6.7 mg of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 220 mg of levetiracetam; b) 280 mg of hydroxypropyl methylcellulose (or hypromellose) Methocel™ K15M CR; c) 1.2 mg of colloidal silicon dioxide; d) 92.8 mg of silicified microcrystalline cellulose ProSolv™ HD90; and e) 6.0 mg of magnesium stearate (*e.g*., HyQual®). In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 220 mg of levetiracetam; b) 347.5 mg of hydroxypropyl methylcellulose (or hypromellose) Methocel™ K15M CR; c) 1.4 mg of colloidal silicon dioxide; d) 119.2 mg of silicified microcrystalline cellulose ProSolv™ HD90; and e) 6.7 mg of magnesium stearate (*e.g*., HyQual®). In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 190 mg of levetiracetam; b) 300 mg of hydroxypropyl methylcellulose (or hypromellose); c) 1.2 mg of colloidal silicon dioxide; d) 102.8 mg of silicified microcrystalline cellulose; and e) 6 mg of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 190 mg of levetiracetam; b) 300 mg of hydroxypropyl methylcellulose (or hypromellose); c) 1.2 mg of colloidal silicon dioxide; d) 102.8 mg of anhydrous dicalcium phosphate; and e) 6 mg of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the magnesium stearate is HyQual®. In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 190 mg of levetiracetam; b) 300 mg of hydroxypropyl methylcellulose (or hypromellose) Methocel™ K15M CR; c) 1.2 mg of colloidal silicon dioxide; d) 102.8 mg of silicified microcrystalline cellulose ProSolv™ HD90; and e) 6 mg of magnesium stearate (*e.g*., HyQual®). In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 190 mg of levetiracetam; b) 300 mg of hydroxypropyl methylcellulose (or hypromellose) Methocel™ K100M Premium CR; c) 1.2 mg of colloidal silicon dioxide; d) 102.8 mg of silicified microcrystalline cellulose ProSolv™ HD90; and e) 6 mg of magnesium stearate (*e.g*., HyQual®). In some embodiments, the extended release pharmaceutical composition is in a solid form. In some embodiments, the extended release pharmaceutical composition is in the form of a tablet or capsule.

In another aspect, the invention provides extended release pharmaceutical compositions comprising: a) 190 mg of levetiracetam; b) 300 mg of hydroxypropyl methylcellulose (or hypromellose) Methocel™ K100M Premium CR; c) 1.2 mg of colloidal silicon dioxide; d) 102.8 mg of anhydrous dicalcium phosphate; and e) 6 mg of magnesium stearate (*e.g*., HyQual®). In some embodiments, the extended release pharmaceutical composition is in a solid form. In some embodiments, the extended release pharmaceutical composition is in the form of a tablet or capsule.

In another aspect, the invention provides extended release pharmaceutical compositions comprising 100-350 mg of levetiracetam, a matrix-forming polymer, a glidant, a diluent, and a lubricant. In some embodiments, the matrix-forming polymer is water soluble. In some embodiments, the diluent is water soluble. In some embodiments, the amount of levetiracetam in the extended release pharmaceutical compositions is 125-250 mg of levetiracetam. In some embodiments, the percentage of the matrix-forming polymer in the extended release pharmaceutical compositions is any range between 45% w/w-70% w/w, such as 45%, 46%, 47%, 48%, 49%, 50%, 55%, 60%, 65%, or 70% w/w.

In another aspect, the invention provides extended release pharmaceutical compositions comprising: a) 30-40% w/w (e.g., 31.7-36.7%w/w) of levetiracetam; b) 45-55% w/w (e.g., 46.7-50%w/w) of hydroxypropyl methylcellulose (or hypromellose); c) 0.01-5% w/w of colloidal silicon dioxide (*e.g.,* 0.2% w/w); d) 15-20% w/w (15.5-17.1%w/w) of silicified microcrystalline cellulose; and e) 0.01-5% w/w of magnesium stearate (*e.g*., 0.96-1%w/w). In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125-250 mg of levetiracetam.

In another aspect, the invention provides extended release pharmaceutical compositions comprising: a) 36.7% w/w of levetiracetam; b) 46.7% w/w of hydroxypropyl methylcellulose (or hypromellose); c) 0.2% w/w of colloidal silicon dioxide; d) 15.5% w/w of silicified microcrystalline cellulose; and e) 1% w/w of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125-250 mg of levetiracetam.

In another aspect, the invention provides extended release pharmaceutical compositions comprising: a) 31.7% w/w of levetiracetam; b) 50% w/w of hydroxypropyl methylcellulose (or hypromellose); c) 0.2% w/w mg of colloidal silicon dioxide; d) 17.1% w/w of silicified microcrystalline cellulose; and e) 0.96% w/w of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 695 mg. In some embodiments, the extended release composition comprises 125-250 mg of levetiracetam.

In another aspect, the invention provides extended release pharmaceutical compositions comprising: a) 36.7% w/w of levetiracetam; b) 46.7% w/w of hydroxypropyl methylcellulose (or hypromellose) Methocel™ K15M CR; c) 0.2% w/w of colloidal silicon dioxide; d) 15.5% w/w of silicified microcrystalline cellulose ProSolv™ HD90; and e) 1% w/w of magnesium stearate (*e.g.*, HyQual®). In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125-250 mg of levetiracetam.

In another aspect, the invention provides extended release pharmaceutical compositions comprising: a) 31.7% w/w of levetiracetam; b) 50% w/w of hydroxypropyl methylcellulose (or hypromellose) Methocel™ K15M CR; c) 0.2% w/w of colloidal silicon dioxide; d) 17.1% w/w of silicified microcrystalline cellulose ProSolv™ HD90; and e) 0.96% w/w of magnesium stearate (*e.g*., HyQual®). In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 695 mg. In some embodiments, the extended release composition comprises 125-250 mg of levetiracetam.

In another aspect, the invention provides extended release pharmaceutical compositions comprising: a) 31.7% w/w of levetiracetam; b) 50% w/w of hydroxypropyl methylcellulose (or hypromellose); c) 0.2% w/w of colloidal silicon dioxide; d) 17.1% w/w of silicified microcrystalline cellulose; and e) 1% w/w of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the silicified microcrystalline cellulose is ProSolv™ HD90. In some embodiments, the magnesium stearate is HyQual®. In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125-250 mg of levetiracetam.

In another aspect, the invention provides extended release pharmaceutical compositions comprising: a) 31.7% w/w of levetiracetam; b) 50% w/w of hydroxypropyl methylcellulose (or hypromellose); c) 0.2% w/w of colloidal silicon dioxide; d) 17.1% w/w of anhydrous dicalcium phosphate; and e) 1% w/w of magnesium stearate. In some embodiments, the hydroxypropyl methylcellulose (or hypromellose) is Methocel™ K15M CR. In some embodiments, the hydroxypropyl methylcellulose (or hypromellose) is Methocel™ K100M Premium CR. In some embodiments, the magnesium stearate is HyQual®. In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125 -250 mg of levetiracetam.

In another aspect, the invention provides extended release pharmaceutical compositions comprising: a) 31.7% w/w of levetiracetam; b) 50% w/w of hydroxypropyl methylcellulose (or hypromellose) Methocel™ K15M CR; c) 0.2% w/w of colloidal silicon dioxide; d) 17.1% w/w of silicified microcrystalline cellulose ProSolv™ HD90; and e) 1% w/w of magnesium stearate (*e.g*., HyQual®). In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125 -250 mg of levetiracetam.

In another aspect, the invention provides extended release pharmaceutical compositions comprising: a) 31.7% w/w of levetiracetam; b) 50% w/w of hydroxypropyl methylcellulose (or hypromellose) Methocel™ K100M Premium CR; c) 0.2% w/w of colloidal silicon dioxide; d) 17.1% w/w of silicified microcrystalline cellulose ProSolv™ HD90; and e) 1% w/w of magnesium stearate (*e.g*., HyQual®). In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125-250 mg of levetiracetam.

In another aspect, the invention provides extended release pharmaceutical compositions comprising: a) 31.7% w/w of levetiracetam; b) 50% w/w of hydroxypropyl methylcellulose (or hypromellose) Methocel™ K100M Premium CR; c) 0.2% w/w of colloidal silicon dioxide; d) 17.1% w/w of anhydrous dicalcium phosphate; and e) 1% w/w of magnesium stearate HyQual®. In some embodiments, the extended release pharmaceutical composition is in a solid form. In some embodiments, the extended release pharmaceutical composition is in the form of a tablet or capsule. In some embodiments, the total weight of the extended release pharmaceutical composition is 250 mg - 1000 mg. In a particular embodiment, the total weight of the extended release pharmaceutical composition is 600 mg. In some embodiments, the extended release composition comprises 125-250 mg of levetiracetam.

In some embodiments, the invention uses hydroxypropyl methylcellulose (or hypromellose) as a rate controlling polymer or a matrix forming polymer in the extended release compositions. In some embodiments, hydroxypropyl methylcellulose (or hypromellose) can be used together with other rate controlling polymers or matrix forming polymers in the compositions of this invention. In some embodiments, hydroxypropyl methylcellulose can be replaced by other rate controlling polymers or matrix forming polymers in the compositions of this invention. In some embodiments, the rate controlling polymers or matrix forming polymers that can replace hydroxypropyl methylcellulose or be used together with hydroxypropyl methylcellulose have similar properties or characteristics as hydroxypropyl methylcellulose. Examples of these rate controlling polymers or matrix forming polymers include, without being limited to, cellulose, non-cellulose polysaccharide, polyvinyl polymer, hydrogel, monolithic polymer, or mixtures thereof. In some embodiments, hydroxypropyl methylcellulose, hydroxyethyl cellulose, hydroxypropyl cellulose, methylcellulose, sodium carboxymethyl cellulose, sodium alginate, carbomer, xanthan gum, guar gum, locust bean gum, carob gum, arabic gum, sterculia gum, polyvinyl pyrrolidone, polyvinyl acetate, polyvinyl alcohol, polyethylene oxide, or a mixture thereof can be used as a rate controlling polymer or matrix forming polymer in the compositions of the invention. In some embodiments, the hydroxypropyl methylcellulose (or hypromellose) in the compositions of the present invention may be selected from the group consisting of Methocel™ K4M, K15M, K100M, E4M, E10M, K4M CR, K15M CR, K100M CR, E4M CR, E10M CR, K4M Premium, K15M Premium, K100M Premium, E4M Premium, E10M Premium, K4M Premium CR, K15M Premium CR, K100M Premium CR, E4M Premium CR, E10M Premium CR, and K100 Premium LV.

In some embodiments, the invention uses colloidal silicon dioxide as a glidant in the extended release compositions. In some embodiments, colloidal silicon dioxide can be used together with other glidants in the compositions of this invention. In some embodiments, colloidal silicon dioxide can be replaced by other glidants in the compositions of this invention. In some embodiments, the glidant that can replace colloidal silicon dioxide or that can be used together with colloidal silicon dioxide have similar properties or characteristics as colloidal silicon dioxide. Examples of these glidants include, without being limited to, cornstarch, talc, calcium silicate, magnesium silicate, aluminum silicate, silicon hydrogel or a mixture thereof.

In some embodiments, the invention uses silicified microcrystalline cellulose as a diluent in the extended release compositions. In some embodiments, silicified microcrystalline cellulose can be used together with other diluents in the compositions of this invention. In some embodiments, silicified microcrystalline cellulose can be replaced by other diluents in the compositions of this invention. In some embodiments, the diluent that can replace silicified microcrystalline cellulose or that can be used together with silicified microcrystalline cellulose have similar properties or characteristics as silicified microcrystalline cellulose, such as water solubility. Examples of these diluents (or water soluble diluents) include, without being limited to, microcrystalline cellulose, lactose, mannitol, xylitol, dextrose, sucrose, sorbitol, compressible sugar, powdered cellulose, cornstarch, pregelatinized starch, dextrate, dextran, dextrin, dextrose, maltodextrin, calcium carbonate, polyethylene oxide, and a mixture thereof.

In some embodiments, the invention uses magnesium stearate as a lubricant in the extended release compositions. In some embodiments, magnesium stearate can be used together with other lubricants in the compositions of this invention. In some embodiments, magnesium stearate can be replaced by other lubricants in the compositions of this invention. In some embodiments, the lubricant that can replace magnesium stearate or that can be used together with magnesium stearate have similar properties or characteristics as magnesium stearate. Examples of these lubricants include, without being limited to, calcium stearate, sodium stearyl fumerate, glyceryl palmitostearate, glyceryl stearate, mineral oil, stearic acid, zinc stearate and a mixture thereof.

The compositions described herein can further contain pharmaceutically acceptable excipient(s) and may contain other agents that serve to enhance and/or complement the effectiveness of the levetiracetam, or to enhance or improve the extended release profile or pharmacokinetic profile of the levetiracetam.

In some embodiments, the pharmaceutical composition of the present invention is the formulations in **Table 1.** In one embodiment, the pharmaceutical composition of the present invention is the 190 mg Tablet A formulation.

In some embodiments, the pharmaceutical composition of the present invention is the formulations in **Table 3.** In one embodiment, the pharmaceutical composition of the present invention is the 220 mg Tablet D formulation.

In some embodiments, the extended release levetiracetam compositions are formulated for once daily administration. For example, the extended release compositions comprise 190 mg of levetiracetam and provide a daily dosage of 190 mg when administered once a day (*i.e.,* every twenty-four hours). The extended release compositions comprise 220 mg of levetiracetam and provide a daily dosage of 220 mg when administered once a day (*i.e.,* every twenty-four hours). In some embodiments, the extended release levetiracetam compositions are formulated for one-unit-dosage-form-once-daily administration. In some embodiments, the extended release levetiracetam compositions are formulated for one-tablet-once-daily administration. For example, subjects who suffer from cognitive impairment will take the extended release composition of the invention (*e.g.*, Tablets A, B, and C in **Table 1** or Tablets D and E in **Table 3**) one-tablet-once-a-day. Each tablet is an extended release dosage form comprising 190 mg or 220 mg of levetiracetam.

In some embodiments, the extended release levetiracetam compositions are administered in the morning.

In some embodiments, the extended release levetiracetam compositions are in a solid form, for example, tablets, capsules, mini-tablets, mini tablets in a capsule, dragrees, pills, lozenges, granules, films or dissolvable films. In some embodiments, the compositions of the present invention are in the form of a tablet. In some embodiments, the tablet is a homogeneous mixture.

In some embodiments, the extended release levetiracetam compositions are formulated for oral administration. In some embodiments, the extended release pharmaceutical compositions are formulated in a solid form for oral administration. In some embodiments, the extended release levetiracetam compositions are oral tablets. In some embodiments, the extended release levetiracetam compositions are oral capsules. In some embodiments, the extended release levetiracetam compositions are formulated for injection or sublingual administration. In some embodiments, the extended release levetiracetam compositions are formulated for administration in the form of a patch or a pump.

In some embodiments, the pharmaceutical compositions of the invention do not comprise a hydrophobic rate controlling polymer.

In some embodiments, the pharmaceutical compositions of the invention do not comprise a functional coating.

### Description of Methods of the Invention

The methods of this invention comprise administration of the extended release compositions of levetiracetam for treating cognitive impairment associated with central nervous system (CNS) disorders in a subject in need or at risk thereof, including, without limitation, subjects having or at risk for age-related cognitive impairment, Mild Cognitive Impairment (MCI), amnestic MCI (aMCI), Age-Associated Memory Impairment (AAMI), Age Related Cognitive Decline (ARCD), dementia, Alzheimer's Disease(AD), prodromal AD, post traumatic stress disorder (PTSD), schizophrenia, bipolar disorder, amyotrophic lateral sclerosis (ALS), cancer-therapy-related cognitive impairment, mental retardation, Parkinson's disease (PD), autism, compulsive behavior, and substance addiction.

In some embodiments, treatment comprises improving cognitive function in patients suffering from or at risk for cognitive impairment associated with a CNS disorder, such as age-related cognitive impairment, Mild Cognitive Impairment (MCI), amnestic MCI (aMCI), Age-Associated Memory Impairment (AAMI), Age Related Cognitive Decline (ARCD), dementia, Alzheimer's Disease(AD), prodromal AD, post traumatic stress disorder (PTSD), schizophrenia, bipolar disorder, amyotrophic lateral sclerosis (ALS), cancer-therapy-related cognitive impairment, mental retardation, Parkinson's disease (PD), autism, compulsive behavior, and substance addiction. In certain embodiments, treatment comprises slowing or delaying the progression of the CNS disorder. In certain embodiments, treatment comprises preventing, slowing, or delaying the progression of cognitive impairment associated with the CNS disorder. In certain embodiments, treatment comprises reducing the rate of decline of cognitive function associated with the CNS disorder. In certain embodiments, treatment comprises alleviation, amelioration or slowing the progression, of one or more symptoms associated with the CNS disorder, such as cognitive impairment.

### Methods of Assessing Cognitive Impairment

Animal models serve as an important resource for developing and evaluating treatments for cognitive impairment associated with CNS disorders. Features that characterize cognitive impairment in animal models typically extend to cognitive impairment in humans. Efficacy in such animal models is, thus, expected to be predictive of efficacy in humans. The extent of cognitive impairment in an animal model for a CNS disorder, and the efficacy of a method of treatment for said CNS disorder may be tested and confirmed with the use of a variety of cognitive tests.

A Radial Arm Maze (RAM) behavioral task is one example of a cognitive test, specifically testing spacial memory (Chappell et al. Neuropharmacology 37: 481-487, 1998). The RAM apparatus consists of, *e.g.,* eight equidistantly spaced arms. A maze arm projects from each facet of a center platform. A food well is located at the distal end of each arm. Food is used as a reward. Blocks can be positioned to prevent entry to any arm. Numerous extra maze cues surrounding the apparatus may also be provided. After habituation and training phases, spatial memory of the subjects may be tested in the RAM under control or test compound-treated conditions. As a part of the test, subjects are pretreated before trials with a vehicle control or one of a range of dosages of the test compound. At the beginning of each trial, a subset of the arms of the eight-arm maze is blocked. Subjects are allowed to obtain food on the unblocked arms to which access is permitted during this initial "information phase" of the trial. Subjects are then removed from the maze for a delay period, *e.g.,* a 60 second delay, a 15 minute delay, a one-hour delay, a two-hour delay, a six hour delay, a 24 hour delay, or longer) between the information phase and the subsequent "retention test," during which the barriers on the maze are removed, thus allowing access to all eight arms. After the delay period, subjects are placed back onto the center platform (with the barriers to the previously blocked arms removed) and allowed to obtain the remaining food rewards during this retention test phase of the trial. The identity and configuration of the blocked arms vary across trials. The number of "errors" the subjects make during the retention test phase is tracked. An error occurs in the trial if the subjects entered an arm from which food had already been retrieved in the pre-delay component of the trial, or if it re-visits an arm in the post-delay session that had already been visited. A fewer number of errors would indicate better spatial memory. The number of errors made by the test subject, under various test compound treatment regimes, can then be compared for efficacy of the test compound in treating cognitive impairment associated with CNS disorders.

Another cognitive test that may be used to assess the effects of a test compound on the cognitive impairment of a CNS disorder model animal is the Morris water maze. A water maze is a pool surrounded with a novel set of patterns relative to the maze. The training protocol for the water maze may be based on a modified water maze task that has been shown to be hippocampal-dependent (de Hoz et al., Eur. J. Neurosci., 22:745-54, 2005; Steele and Morris, Hippocampus 9:118-36, 1999). The subject is trained to locate a submerged escape platform hidden underneath the surface of the pool. During the training trial, a subject is released in the maze (pool) from random starting positions around the perimeter of the pool. The starting position varies from trial to trial. If the subject does not locate the escape platform within a set time, the experimenter guides and places the subject on the platform to "teach" the location of the platform. After a delay period following the last training trial, a retention test in the absence of the escape platform is given to assess spatial memory. The subject's level of preference for the location of the (now absent) escape platform, as measured by, *e.g.,* the time spent in that location or the number of crossings of that location made by the mouse, indicates better spatial memory, *i.e.,* treatment of cognitive impairment. The preference for the location of the escape platform under different treatment conditions, can then be compared for efficacy of the test compound in treating cognitive impairment associated with CNS disorders.

There are various tests known in the art for assessing cognitive function in humans, for example and without limitation, the clinical global impression of change scale (CIBIC-plus scale); the Mini Mental State Exam (MMSE); the Neuropsychiatric Inventory (NPI); the Clinical Dementia Rating Scale (CDR); the Cambridge Neuropsychological Test Automated Battery (CANTAB); the Sandoz Clinical Assessment-Geriatric (SCAG), the Buschke Selective Reminding Test (Buschke and Fuld, 1974); the Verbal Paired Associates subtest; the Logical Memory subtest; the Visual Reproduction subtest of the Wechsler Memory Scale-Revised (WMS-R) (Wechsler, 1997); the Benton Visual Retention Test, or MATRICS consensus neuropsychological test battery which includes tests of working memory, speed of processing, attention, verbal learning, visual learning, reasoning and problem solving and social cognition. *See* Folstein et al., J Psychiatric Res 12: 189-98, (1975); Robbins et al., Dementia 5: 266-81, (1994); Rey, L'examen clinique en psychologie, (1964); Kluger et al., J Geriatr Psychiatry Neurol 12:168-79, (1999); Marquis et al., 2002 and Masur et al., 1994, or MATRICS consensus neuropsychological test battery which includes tests of working memory, speed of processing, attention, verbal learning, visual learning, reasoning and problem solving and social cognition. Another example of a cognitive test in humans is the explicit 3-alternative forced choice task. In this test, subjects are presented with color photographs of common objects consisting of a mix of three types of image pairs: similar pairs, identical pairs and unrelated foils. The second of the pair of similar objects is referred to as the "lure". These image pairs are fully randomized and presented individually as a series of images. Subjects are instructed to make a judgment as to whether the objects seen are new, old or similar. A "similar" response to the presentation of a lure stimulus indicates successful memory retrieval by the subject. By contrast, calling the lure stimulus "old" or "new" indicates that correct memory retrieval does not occur.

In addition to assessing cognitive performance, the progression of age-related cognitive impairment and dementia, as well as the conversion of age-related cognitive impairment into dementia, may be monitored by assessing surrogate changes in the brain of the subject. Surrogate changes include, without limitation, changes in regional brain volumes, perforant path degradation, and changes seen in brain function through resting state fMRI (R-fMRI) and fluorodeoxyglucose positron emission tomography (FDG-PET). Examples of regional brain volumes useful in monitoring the progression of age-related cognitive impairment and dementia include reduction of hippocampal volume and reduction in volume or thickness of entorhinal cortex. These volumes may be measured in a subject by, for example, MRI. Aisen et al., Alzheimer's & Dementia 6:239-246 (2010). Perforant path degradation has been shown to be linked to age, as well as reduced cognitive function. For example, older adults with more perforant path degradation tend to perform worse in hippocampus-dependent memory tests. Perforant path degradation may be monitored in subjects through ultrahigh-resolution diffusion tensor imaging (DTI). Yassa et al., PNAS 107:12687-12691 (2010). Resting-state fMRI (R-fMRI) involves imaging the brain during rest, and recording large-amplitude spontaneous low-frequency (<0.1 Hz) fluctuations in the fMRI signal that are temporally correlated across functionally related areas. Seed-based functional connectivity, independent component analyses, and/or frequency-domain analyses of the signals are used to reveal functional connectivity between brain areas, particularly those areas whose connectivity increase or decrease with age, as well as the extent of cognitive impairment and/or dementia. FDG-PET uses the uptake of FDG as a measure of regional metabolic activity in the brain. Decline of FDG uptake in regions such as the posterior cingulated cortex, temporoparietal cortex, and prefrontal association cortex has been shown to relate to the extent of cognitive decline and dementia. Aisen et al., Alzheimer's & Dementia 6:239-246 (2010), Herholz et al., NeuroImage 17:302-316 (2002).

### Age-Related Cognitive Impairment

This invention provides methods for treating age-related cognitive impairment or the risk thereof using the extended release levetiracetam compositions of the invention. In certain embodiments, treatment comprises improving cognitive function in patients with age-related cognitive impairment. In certain embodiments, treatment comprises slowing or delaying the progression of age-related cognitive impairment. In certain embodiments, treatment comprises reducing the rate of decline of cognitive function associated with age-related cognitive impairment. In certain embodiments, treatment comprises preventing or slowing the progression, of age-related cognitive impairment. In certain embodiments, treatment comprises alleviation, amelioration or slowing the progression, of one or more symptoms associated with age-related cognitive impairment. In certain embodiments, treatment of age-related cognitive impairment comprises slowing the conversion of age-related cognitive impairment (including, but not limited to MCI, ARCD and AAMI) into dementia (e.g., AD). The methods and compositions may be used for human patients in clinical applications in the treating age-related cognitive impairment in conditions such as MCI, ARCD and AAMI or for the risk thereof. The dose of the composition and dosage interval for the method is, as described herein, one that is safe and efficacious in those applications.

In some embodiments, a subject to be treated by the methods and compositions of this invention exhibits age-related cognitive impairment or is at risk of such impairment. In some embodiments, the age-related cognitive impairment includes, without limitation, Age-Associated Memory Impairment (AAMI), Mild Cognitive Impairment (MCI) and Age-related Cognitive Decline (ARCD).

Animal models serve as an important resource for developing and evaluating treatments for such age-related cognitive impairments. Features that characterize age-related cognitive impairment in animal models typically extend to age-related cognitive impairment in humans. Efficacy in such animal models is, thus, expected to be predictive of efficacy in humans.

Various animal models of age-related cognitive impairment are known in the art. For example, extensive behavioral characterization has identified a naturally occurring form of cognitive impairment in an outbred strain of aged Long-Evans rats (Charles River Laboratories; Gallagher et al., Behav. Neurosci. 107:618-626, (1993)). In a behavioral assessment with the Morris Water Maze (MWM), rats learn and remember the location of an escape platform guided by a configuration of spatial cues surrounding the maze. The cognitive basis of performance is tested in probe trials using measures of the animal's spatial bias in searching for the location of the escape platform. Aged rats in the study population have no difficulty swimming to a visible platform, but an age-dependent impairment is detected when the platform is camouflaged, requiring the use of spatial information. Performance for individual aged rats in the outbred Long-Evans strain varies greatly. For example, a proportion of those rats perform on a par with young adults. However, approximately 40-50% fall outside the range of young performance. This variability among aged rats reflects reliable individual differences. Thus, within the aged population some animals are cognitively impaired and designated aged-impaired (AI) and other animals are not impaired and are designated aged-unimpaired (AU). *See, e.g.,* Colombo et al., Proc. Natl. Acad. Sci. 94: 14195-14199, (1997); Gallagher and Burwell, Neurobiol. Aging 10: 691-708, (1989); Gallagher et al. Behav. Neurosci. 107:618-626, (1993); Rapp and Gallagher, Proc. Natl. Acad. Sci. 93: 9926-9930, (1996); Nicolle et al., Neuroscience 74: 741-756, (1996); Nicolle et al., J. Neurosci. 19: 9604-9610, (1999); International Patent Publication WO2007/019312 and International Patent Publication WO 2004/048551. Such an animal model of age-related cognitive impairment may be used to assay the effectiveness of the methods and compositions this invention in treating age-related cognitive impairment.

The efficacy of the methods and compositions of this invention in treating age-related cognitive impairment may be assessed using a variety of cognitive tests, including the Morris water maze and the radial arm maze, as discussed above.

### Dementia

This invention also provides methods for treating dementia using the extended release levetiracetam compositions of the invention. In certain embodiments, treatment comprises improving cognitive function in patients with dementia. In certain embodiments, treatment comprises slowing or delaying the progression of dementia. In certain embodiments, treatment comprises reducing the rate of decline of cognitive function associated with dementia. In certain embodiments, treatment comprises preventing or slowing the progression, of dementia. In certain embodiments, treatment comprises alleviation, amelioration, or slowing the progression of one or more symptoms associated with dementia. In certain embodiments, the symptom to be treated is cognitive impairment. In certain embodiments, the dementia is Alzheimer's disease (AD), vascular dementia, dementia with Lewy bodies, or frontotemporal dementia. The methods and compositions may be used for human patients in clinical applications in treating dementia. The dose of the composition and dosage interval for the method is, as described herein, one that is safe and efficacious in those applications.

Animal models serve as an important resource for developing and evaluating treatments for dementia. Features that characterize dementia in animal models typically extend to dementia in humans. Thus, efficacy in such animal models is expected to be predictive of efficacy in humans. Various animal models of dementia are known in the art, such as the PDAPP, Tg2576, APP23, TgCRND8, J20, hPS2 Tg, and APP + PS1 transgenic mice. Sankaranarayanan, Curr. Top. Medicinal Chem. 6: 609-627, 2006; Kobayashi et al. Genes Brain Behav. 4: 173-196. 2005; Ashe and Zahns, Neuron. 66: 631-45, 2010. Such animal models of dementia may be used to assay the effectiveness of the methods and compositions of this invention of the invention in treating dementia.

The efficacy of the methods and compositions of this invention in treating dementia, or cognitive impairment associated with dementia, may be assessed in animals models of dementia, as well as human subjects with dementia, using a variety of cognitive tests known in the art, as discussed above.

### Post Traumatic Stress Disorder

This invention also provides methods for treating post traumatic stress disorder (PTSD) using the extended release levetiracetam compositions of the invention. In certain embodiments, treatment comprises improving cognitive function in patients with PTSD. In certain embodiments, treatment comprises slowing or delaying the progression of PTSD. In certain embodiments, treatment comprises reducing the rate of decline of cognitive function associated with PTSD. In certain embodiments, treatment comprises preventing or slowing the progression, of PTSD. In certain embodiments, treatment comprises alleviation, amelioration, or slowing the progression of one or more symptoms associated with PTSD. In certain embodiments, the symptom to be treated is cognitive impairment. The methods and compositions may be used for human patients in clinical applications in treating PTSD. The dose of the composition and dosage interval for the method is, as described herein, one that is safe and efficacious in those applications.

Patients with PTSD (and, to a lesser degree trauma-exposed patients without PTSD) have smaller hippocampal volumes (Woon et al., Prog. Neuro-Psychopharm. & Biological Psych. 34, 1181-1188; Wang et al., Arch. Gen. Psychiatry 67:296-303, 2010). PTSD is also associated with impaired cognitive performance. Older individuals with PTSD have greater declines in cognitive performance relative to control patients (Yehuda et al., Bio. Psych. 60: 714-721, 2006) and have a greater likelihood of developing dementia (Yaffe et al., Arch. Gen. Psych. 678: 608-613, 2010).

Animal models serve as an important resource for developing and evaluating treatments for PTSD. Features that characterize PTSD in animal models typically extend to PTSD in humans. Thus, efficacy in such animal models is expected to be predictive of efficacy in humans. Various animal models of PTSD are known in the art.

One rat model of PTSD is Time-dependent sensitization (TDS). TDS involves exposure of the animal to a severely stressful event followed by a situational reminder of the prior stress. The following is an example of TDS. Rats are placed in a restrainer, then placed in a swim tank and made to swim for a period of time, *e.g.,* 20 min. Following this, each rat is then immediately exposed to a gaseous anesthetic until loss of consciousness, and finally dried. The animals are left undisturbed for a number of days, *e.g.,* one week. The rats are then exposed to a "restress" session consisting of an initial stressor, *e.g.,* a swimming session in the swim tank (Liberzon et al., Psychoneuroendocrinology 22: 443-453, 1997; Harvery et al., Psychopharmacology 175:494-502, 2004). TDS results in an enhancement of the acoustic startle response (ASR) in the rat, which is comparable to the exaggerated acoustic startle that is a prominent symptom of PTSD (Khan and Liberzon, Psychopharmacology 172: 225-229, 2004). Such animal models of PTSD may be used to assay the effectiveness of the methods and compositions of this invention of the invention in treating PTSD.

The efficacy of the methods and compositions of this invention in treating PTSD, or cognitive impairment associated with PTSD, may also be assessed in animals models of PTSD, as well as human subjects with PTSD, using a variety of cognitive tests known in the art, as discussed above.

### Schizophrenia

This invention provides methods for treating schizophrenia or bipolar disorder (in particular, mania) using the extended release levetiracetam compositions of the invention. In certain embodiments, treatment comprises improving cognitive function in patients with schizophrenia. In certain embodiments, treatment comprises slowing or delaying the progression of schizophrenia. In certain embodiments, treatment comprises reducing the rate of decline of cognitive function associated with schizophrenia. In certain embodiments, treatment comprises preventing or slowing the progression of schizophrenia or bipolar disorder (in particular, mania). Schizophrenia is characterized by a wide spectrum of psychopathology, including positive symptoms such as aberrant or distorted mental representations (*e.g*., hallucinations, delusions), negative symptoms characterized by diminution of motivation and adaptive goal-directed action (*e.g.*, anhedonia, affective flattening, avolition), and cognitive impairment. In certain embodiments, treatment comprises alleviation, amelioration or slowing the progression of one or more positive and/or negative symptoms, as well as cognitive impairment, associated with schizophrenia. Further, there are a number of other psychiatric diseases such as schizotypical and schizoaffective disorder, other acute- and chronic psychoses and bipolar disorder (in particular, mania), which have an overlapping symptomatology with schizophrenia. In some embodiments, treatment comprises alleviation, amelioration or slowing the progression of one or more symptoms, as well as cognitive impairment, associated with bipolar disorder (in particular, mania). The methods and compositions may be used for human patients in clinical applications in treating schizophrenia or bipolar disorder (in particular, mania). The dose of the composition and dosage interval for the method is, as described herein, one that is safe and efficacious in those applications.

Cognitive impairments are associated with schizophrenia. They precede the onset of psychosis and are present in non-affected relatives. The cognitive impairments associated with schizophrenia constitute a good predictor for functional outcome and are a core feature of the disorder. Cognitive features in schizophrenia reflect dysfunction in frontal cortical and hippocampal circuits. Patients with schizophrenia also present hippocampal pathologies such as reductions in hippocampal volume, reductions in neuronal size and dysfunctional hyperactivity. An imbalance in excitation and inhibition in these brain regions has also been documented in schizophrenic patients suggesting that drugs targeting inhibitory mechanisms could be therapeutic. *See, e.g.,* Guidotti et al., Psychopharmacology 180: 191-205, 2005; Zierhut, Psych. Res. Neuroimag. 183:187-194, 2010; Wood et al., Neurolmage 52:62-63, 2010; Vinkers et al., Expert Opin. Investig. Drugs 19:1217-1233, 2009; Young et al., Pharmacol. Ther. 122:150-202, 2009.

Animal models serve as an important resource for developing and evaluating treatments for schizophrenia. Features that characterize schizophrenia in animal models typically extend to schizophrenia in humans. Thus, efficacy in such animal models is expected to be predictive of efficacy in humans. Various animal models of schizophrenia are known in the art.

One animal model of schizophrenia is protracted treatment with methionine. Methionine-treated mice exhibit deficient expression of GAD67 in frontal cortex and hippocampus, similar to those reported in the brain of postmortem schizophrenia patients. They also exhibit prepulse inhibition of startle and social interaction deficits (Tremonlizzo et al., PNAS, 99: 17095-17100, 2002). Another animal model of schizophrenia is methylaoxymethanol acetate (MAM)-treatment in rats. Pregnant female rats are administered MAM (20 mg/kg, intraperitoneal) on gestational day 17. MAM-treatment recapitulate a pathodevelopmental process to schizophrenia-like phenotypes in the offspring, including anatomical changes, behavioral deficits and altered neuronal information processing. More specifically, MAM-treated rats display a decreased density of parvalbumin-positive GABAergic interneurons in portions of the prefrontal cortex and hippocampus. In behavioral tests, MAM-treated rats display reduced latent inhibition. Latent inhibition is a behavioral phenomenon where there is reduced learning about a stimulus to which there has been prior exposure with any consequence. This tendency to disregard previously benign stimuli, and reduce the formation of association with such stimuli is believed to prevent sensory overload. Low latent inhibition is indicative of psychosis. Latent inhibition may be tested in rats in the following manner. Rats are divided into two groups. One group is pre-exposed to a tone over multiple trials. The other group has no tone presentation. Both groups are then exposed to an auditory fear conditioning procedure, in which the same tone is presented concurrently with a noxious stimulus, *e.g*. an electric shock to the foot. Subsequently, both groups are presented with the tone, and the rats' change in locomotor activity during tone presentation is monitored. After the fear conditioning the rats respond to the tone presentation by strongly reducing locomotor activity. However, the group that has been exposed to the tone before the conditioning period displays robust latent inhibition: the suppression of locomotor activity in response to tone presentation is reduced. MAM-treated rats, by contrast show impaired latent inhibition. That is, exposure to the tone previous to the fear conditioning procedure has no significant effect in suppressing the fear conditioning. *(see* Lodge et al., J. Neurosci., 29:2344-2354, 2009) Such animal models of schizophrenia may be used to assay the effectiveness of the methods and compositions of the invention in treating schizophrenia or bipolar disorder (in particular, mania).

MAM-treated rats display a significantly enhanced locomotor response (or aberrant locomotor activity) to low dose D-amphetamine administration. The MAM-treated rats also display a significantly greater number of spontaneously firing ventral tegmental dopamine (DA) neurons. These results are believed to be a consequence of excessive hippocampal activity because in MAM-treated rats, the ventral hippocampus (vHipp) inactivation (*e.g*., by intra-vHipp administration of a sodium channel blocker, tetrodotoxin (TTX), to MAM rats) completely reversed the elevated DA neuron population activity and also normalized the augmented amphetamine-induced locomotor behavior. The correlation of hippocampal dysfunction and the hyper-responsivity of the DA system is believed to underlie the augmented response to amphetamine in MAM-treated animals and psychosis in schizophrenia patients. See Lodge D. J. et al. Neurobiology of Disease (2007), 27(42), 11424-11430. The use of MAM-treated rats in the above study may be suitable for use to assay the effectiveness of the methods and compositions of the present invention in treating schizophrenia or bipolar disorder (in particular, mania). For example, the methods and compositions of this invention maybe evaluated, using MAM-treated animals, for their effects on the central hippocampus (vHipp) regulation, on the elevated DA neuron population activity and on the hyperactive locomotor response to amphetamine in the MAM-treated animals.

In MAM-treated rats, hippocampal (HPC) dysfunction leads to dopamine system hyperactivity. A benzodiazepine-positive allosteric modulator (PAM), selective for the α5 subunit of the GABA_{A} receptor, SH-053-2'F-R-CH₃, is tested for its effects on the output of the hippocampal (HPC). The effect of SH-053-2'F-R-CH₃ on the hyperactive locomotor response to amphetamine in MAM-treated animals is also examined. The α5GABAAR PAM reduces the number of spontaneously active DA neurons in the ventral tegmental area (VTA) of MAM rats to levels observed in saline-treated rats (control group), both when administered systemically and when directly infused into the ventral HPC. Moreover, HPC neurons in both saline-treated and MAM-treated animals show diminished cortical-evoked responses following the α5GABAAR PAM treatment. In addition, the increased locomotor response to amphetamine observed in MAM-treated rats is reduced following the α5GABA_{A}R PAM treatment. See Gill K. M et al. Neuropsychopharmacology (2011), 1-9. The use of MAM-treated rats in the above study may be suitable for use in the present invention to assay the effectiveness of the methods and compositions of the invention in treating schizophrenia or bipolar disorder (in particular, mania). For example, the methods and compositions of this invention maybe evaluated, using MAM-treated animals, for their effects on the output of the hippocampal (HPC) and on the hyperactive locomotor response to amphetamine in the MAM-treated animals.

Administration of MAM to pregnant rats on embryonic day 15 (E15) severely impairs spatial memory or the ability to learn the spatial location of four items on an eight-arm radial maze in the offspring. In addition, embryonic day 17 (E17) MAM-treated rats are able to reach the level of performance of control rats at the initial stages of training, but are unable to process and retrieve spatial information when a 30-min delay is interposed, indicating a significant impairment in working memory. See Gourevitch R. et al. (2004). Behav. Pharmacol, 15, 287-292. Such animal models of schizophrenia may be used to assay the effectiveness of the methods and compositions of the invention in treating schizophrenia or bipolar disorder (in particular, mania).

Apomorphine-induced climbing (AIC) and stereotype (AIS) in mice is another animal model useful in this invention. Agents are administered to mice at a desired dose level *(e.g.,* via intraperitoneal administration). Subsequently, *e.g.,* thirty minutes later, experimental mice are challenges with apomorphine (e.g., with 1 mg/kg sc). Five minutes after the apomorphine injection, the sniffing-licking-gnawing syndrome (stereotyped behavior) and climbing behavior induced by apomorphine are scored and recorded for each animal. Readings can be repeated every 5 min during a 30-min test session. Scores for each animal are totaled over the 30-min test session for each syndrome (stereotyped behavior and climbing). If an effect reached at least of 50% inhibition, and ID₅₀ value (95% confidence interval) is calculated using a nonlinear least squares calculation with inverse prediction. Mean climbing and stereotype scores can be expressed as a percent of control values observed in vehible treated (*e.g*., saline-treated) mice that receive apomorphine. See Grauer S. M. et al. Psychopharmacology (2009) 204, 37-48. This mice model may be used to assay the effectiveness of the methods and compositions of the invention in treating schizophrenia or bipolar disorder (in particular, mania).

The efficacy of the methods and compositions of this invention in treating schizophrenia may also be assessed in animal models of schizophrenia or bipolar disorder (in particular, mania), as well as human subjects with schizophrenia, using a variety of cognitive tests known in the art, as discussed above.

### Amyotrophic Lateral Sclerosis (ALS)

This invention additionally provides methods for treating ALS using the extended release levetiracetam compositions of the invention. In certain embodiments, treatment comprises improving cognitive function in patients with ALS. In certain embodiments, treatment comprises slowing or delaying the progression of ALS. In certain embodiments, treatment comprises reducing the rate of decline of cognitive function associated with ALS. In certain embodiments, treatment comprises preventing or slowing the progression, of ALS. In certain embodiments, treatment comprises alleviation, amelioration or slowing the progression, of one or more symptoms associated with ALS. In certain embodiments, the symptom to be treated is cognitive impairment. The methods and compositions may be used for human patients in clinical applications in treating ALS. The dose of the composition and dosage interval for the method is, as described herein, one that is safe and efficacious in those applications.

In addition to the degeneration of motor neurons, ALS is characterized by neuronal degeneration in the entorhinal cortex and hippocampus, memory deficits, and neuronal hyperexcitability in different brain areas such as the cortex.

The efficacy of the methods and compositions of this invention in treating ALS, or cognitive impairment associated with ALS, may also be assessed in animal models of ALS, as well as human subjects with ALS, using a variety of cognitive tests known in the art, as discussed above.

### Cancer therapy-related cognitive impairment

This invention additionally provides methods for treating cancer therapy-related cognitive impairment using the extended release levetiracetam compositions of the invention. In certain embodiments, treatment comprises improving cognitive function in patients with cancer therapy-related cognitive impairment. In certain embodiments, treatment comprises slowing or delaying the progression of cancer therapy-related cognitive impairment. In certain embodiments, treatment comprises reducing the rate of decline of cognitive function associated with cancer therapy-related cognitive impairment. In certain embodiments, treatment comprises preventing or slowing the progression, of cancer therapy-related cognitive impairment. In certain embodiments, treatment comprises alleviation, amelioration or slowing the progression, of one or more symptoms associated with cancer therapy-related cognitive impairment. The methods and compositions may be used for human patients in clinical applications in treating cancer therapy-related cognitive impairment. The dose of the composition and dosage interval for the method is, as described herein, one that is safe and efficacious in those applications.

Therapies that are used in cancer treatment, including chemotherapy, radiation, or combinations thereof, can cause cognitive impairment in patients, in such functions as memory, learning and attention. Cytotoxicity and other adverse side-effects on the brain of cancer therapies are the basis for this form of cognitive impairment, which can persist for decades. (Dietrich et al., Oncologist 13:1285-95, 2008; Soussain et al., Lancet 374:1639-51, 2009).

Cognitive impairment following cancer therapies reflects dysfunction in frontal cortical and hippocampal circuits that are essential for normal cognition. In animal models, exposure to either chemotherapy or radiation adversely affects performance on tests of cognition specifically dependent on these brain systems, especially the hippocampus (Kim et al., J. Radiat. Res. 49:517-526, 2008; Yang et al., Neurobiol. Learning and Mem. 93:487-494, 2010). Thus, drugs targeting these cortical and hippocampal systems could be neuroprotective in patients receiving cancer therapies and efficacious in treating symptoms of cognitive impairment that may last beyond the interventions used as cancer therapies.

Animal models serve as an important resource for developing and evaluating treatments for cancer therapy-related cognitive impairment. Features that characterize cancer therapy-related cognitive impairment in animal models typically extend to cancer therapy-related cognitive impairment in humans. Thus, efficacy in such animal models is expected to be predictive of efficacy in humans. Various animal models of cancer therapy-related cognitive impairment are known in the art.

Examples of animal models of cancer therapy-related cognitive impairment include treating animals with anti-neoplastic agents such as cyclophosphamide (CYP) or with radiation, *e.g.,* ⁶⁰Co gamma-rays. (Kim et al., J. Radiat. Res. 49:517-526, 2008; Yang et al., Neurobiol. Learning and Mem. 93:487-494, 2010). The cognitive function of animal models of cancer therapy-related cognitive impairment may then be tested with cognitive tests to assay the effectiveness of the methods and compositions of the invention in treating cancer therapy-related cognitive impairment. The efficacy of the methods and compositions of this invention in treating cancer therapy-related cognitive impairment, as well as human subjects with cancer therapy-related cognitive impairment, using a variety of cognitive tests known in the art, as discussed above.

### Parkinson's disease (PD)

Parkinson's disease (PD) is a neurological disorder characterized by a decrease of voluntary movements. The afflicted patient has reduction of motor activity and slower voluntary movements compared to the normal individual. The patient has characteristic "mask" face, a tendency to hurry while walking, bent over posture and generalized weakness of the muscles. There is a typical "lead-pipe" rigidity of passive movements. Another important feature of the disease is the tremor of the extremities occurring at rest and decreasing during movements.

Parkinson's disease, the etiology of which is unknown, belongs to a group of the most common movement disorders named parkinsonism, which affects approximately one person per one thousand. These other disorders grouped under the name of parkinsonism may result from viral infection, syphilis, arteriosclerosis and trauma and exposure to toxic chemicals and narcotics. Nonetheless, it is believed that the inappropriate loss of synaptic stability may lead to the disruption of neuronal circuits and to brain diseases. Whether as the result of genetics, drug use, the aging process, viral infections, or other various causes, dysfunction in neuronal communication is considered the underlying cause for many neurologic diseases, such as PD (Myrrhe van Spronsen and Casper C. Hoogenraad, Curr. Neurol. Neurosci. Rep. 2010, 10, 207-214).

Regardless of the cause of the disease, the main pathologic feature is degeneration of dopaminergic cells in basal ganglia, especially in substantia nigra. Due to premature death of the dopamine containing neurons in substantia nigra, the largest structure of the basal ganglia, the striatum, will have reduced input from substantia nigra resulting in decreased dopamine release. The understanding of the underlying pathology led to the introduction of the first successful treatment which can alleviate Parkinson's disease. Virtually all approaches to the therapy of the disease are based on dopamine replacement. Drugs currently used in the treatment can be converted into dopamine after crossing the blood brain barrier, or they can boost the synthesis of dopamine and reduce its breakdown. Unfortunately, the main pathologic event, degeneration of the cells in substantia nigra, is not helped. The disease continues to progress and frequently after a certain length of time, dopamine replacement treatment will lose its effectiveness.

This invention provides methods for treating PD using the extended release levetiracetam composition of the invention. In certain embodiments, treatment comprises preventing or slowing the progression of PD. In certain embodiments, treatment comprises alleviation, amelioration, or slowing the progression of one or more symptoms associated with PD. In certain embodiments, the symptom to be treated is cognitive impairment. For example, methods and compositions of the disclosure can be used to improve the motor/cognitive impairments symptomatic of Parkinson's disease. Moreover, methods and compositions of the disclosure may be useful for treating the memory impairment symptomatic of Parkinson's disease.

There are a number of animal models for PD. Exemplary animal models for PD include the reserpine model, the methamphetamine model, the 6-hydroxydopamine (6-OHDA) model, the 1-methyl-4-phenyl-1,2,3,6-tetrahydropyridine (MPTP) model, the paraquat (PQ)-Maneb model, the rotenone model, the 3-nitrotyrosine model and genetic models using transgenic mice. Transgenic models include mice that over express α-synuclein, express human mutant forms of α -synuclein, or mice that express LRKK2 mutations. See review of these models by Ranjita B. et al. (Ranjita B. et al. BioEssays 2002, 24, 308-318). Additional information regarding these animal models is readily available from Jackson Laboratories (see also http://research.jax.org/grs/parkinsons.html), as well as in numerous publications disclosing the use of these validated models.

The efficacy of the methods and compositions of this invention in treating PD, or cognitive impairment associated with PD, may be assessed in any of the above animal models of PD, as well as human subjects with PD, using a variety of cognitive tests known in the art, as discussed above.

### Autism

"Autism", as used herein, refers to an autism spectrum disorder characterized by a neural development disorder leading to impaired social interaction and communication by restricted and repetitive behavior. "Autism Spectrum Disorder" refers to a group of developmental disabilities that includes: autism; Asperger syndrome; pervasive developmental disorder not otherwise specified (PDD-NOS or atypical autism); Rett syndrome; and childhood disintegrative disorder.

Autism is a neurodevelopmental disorder characterized by dysfunction in three core behavioral dimensions: repetitive behaviors, social deficits, and cognitive deficits. The repetitive behavior domain involves compulsive behaviors, unusual attachments to objects, rigid adherence to routines or rituals, and repetitive motor mannerisms such as stereotypies and self- stimulatory behaviors. The social deficit dimension involves deficits in reciprocal social interactions, lack of eye contact, diminished ability to carry on conversation, and impaired daily interaction skills. The cognitive deficits can include language abnormalities. Autism is a disabling neurological disorder that affects thousands of Americans and encompasses a number of subtypes, with various putative causes and few documented ameliorative treatments. The disorders of the autistic spectrum may be present at birth, or may have later onset, for example, at ages two or three. There are no clear cut biological markers for autism. Diagnosis of the disorder is made by considering the degree to which the child matches the behavioral syndrome, which is characterized by poor communicative abilities, peculiarities in social and cognitive capacities, and maladaptive behavioral patterns. The dysfunction in neuronal communication is considered one of the underlying causes for autism (Myrrhe van Spronsen and Casper C. Hoogenraad, Curr. Neurol. Neurosci. Rep. 2010, 10, 207-214).

This invention provides methods for treating autism using the extended release levetiracetam composition of the invention. In certain embodiments, treatment comprises preventing or slowing the progression of autism. In certain embodiments, treatment comprises alleviation, amelioration, or slowing the progression of one or more symptoms associated with autism. In certain embodiments, the symptom to be treated is cognitive deficit. For example, methods and compositions of the disclosure can be used to improve the motor/cognitive deficits symptomatic of autism.

### Mental retardation

Mental retardation is a generalized disorder characterized by significantly impaired cognitive function and deficits in adaptive behaviors. Mental retardation is often defined as an Intelligence Quotient (IQ) score of less than 70. Inborn causes are among many underlying causes for mental retardation. The dysfunction in neuronal communication is also considered one of the underlying causes for mental redardation (Myrrhe van Spronsen and Casper C. Hoogenraad, Curr. Neurol. Neurosci. Rep. 2010, 10, 207-214).

In some instances, mental retardation includes, but are not limited to, Down syndrome, velocariofacial syndrome, fetal alcohol syndrome, Fragile X syndrome, Klinefelter's syndrome, neurofibromatosis, congenital hypothyroidism, Williams syndrome, phenylketonuria (PKU), Smith-Lemli-Opitz syndrome, Prader-Willi syndrome, Phelan-McDermid syndrome, Mowat-Wilson syndrome, ciliopathy, Lowe syndrome and siderium type X-linked mental retardation. Down syndrome is a disorder that includes a combination of birth defects, including some degree of mental retardation, characteristic facial features and, often, heart defects, increased infections, problems with vision and hearing, and other health problems. Fragile X syndrome is a prevalent form of inherited mental retardation, occurring with a frequency of 1 in 4,000 males and 1 in 8,000 females. The syndrome is also characterized by developmental delay, hyperactivity, attention deficit disorder, and autistic-like behavior. There is no effective treatment for fragile X syndrome.

The present invention contemplates the treatment of mild mental retardation, moderate mental retardation, severe mental retardation, profound mental retardation, and mental retardation severity unspecified. Such mental retardation may be, but is not required to be, associated with chromosomal changes, (for example Down Syndrome due to trisomy 21), heredity, pregnancy and perinatal problems, and other severe mental disorders. This invention provides methods for treating mental retardation using the extended release levetiracetam composition of the invention. In certain embodiments, treatment comprises preventing or slowing the progression of mental retardation. In certain embodiments, treatment comprises alleviation, amelioration, or slowing the progression of one or more symptoms associated with mental retardation. In certain embodiments, the symptom to be treated is cognitive deficit/impairment. For example, methods and compositions of the disclosure can be used to improve the motor/cognitive impairments symptomatic of mental retardation.

Several animal models have been developed for mental retardation. For example, a knockout mouse model has been developed for Fragile X syndrome. Fragile X syndrome is a common form of mental retardation caused by the absence of the FMR1 protein, FMRP. Two homologs of FMRP have been identified, FXR1P and FXR2P. FXR2P shows high expression in brain and testis, like FMRP. Both *Fxr2* and *Fmr1* knockout mice, and *Fmr1*/*Fxr2* double knockout mice are believed to be useful models for mental retardation such as Fragile X syndrome. See, Bontekoe C. J. M. et al. Hum. Mol. Genet. 2002, 11 (5): 487-498. The efficacy of the methods and compositions of this invention in treating mental retardation, or cognitive deficit/impairment associated with mental retardation, may be assessed in the these mouse models and other animal models developed for mental retardation, as well as human subjects with mental retardation, using a variety of cognitive tests known in the art, as discussed above.

### Compulsive behavior (obsessive compulsive disorder)

Obsessive compulsive disorder ("OCD") is a mental condition that is most commonly characterized by intrusive, repetitive unwanted thoughts (obsessions) resulting in compulsive behaviors and mental acts that an individual feels driven to perform (compulsion). Current epidemiological data indicates that OCD is the fourth most common mental disorder in the United States. Some studies suggest the prevalence of OCD is between one and three percent, although the prevalence of clinically recognized OCD is much lower, suggesting that many individuals with the disorder may not be diagnosed. Patients with OCD are often diagnosed by a psychologist, psychiatrist, or psychoanalyst according to the Diagnostic and Statistical Manual of Mental Disorders, 4th edition text revision (DSM-IV-TR) (2000) diagnostic criteria that include characteristics of obsessions and compulsions. Characteristics of obsession include: (1) recurrent and persistent thoughts, impulses, or images that are experienced as intrusive and that cause marked anxiety or distress; (2) the thoughts, impulses, or images are not simply excessive worries about real-life problems; and (3) the person attempts to ignore or suppress such thoughts, impulses, or images, or to neutralize them with some other thought or action. The person recognizes that the obsessional thoughts, impulses, or images are a product of his or her own mind, and are not based in reality. Characteristics of compulsion include: (1) repetitive behaviors or mental acts that the person feels driven to perform in response to an obsession, or according to rules that must be applied rigidly; (2) the behaviors or mental acts are aimed at preventing or reducing distress or preventing some dreaded event or situation; however, these behaviors or mental acts are not actually connected to the issue, or they are excessive.

Individuals with OCD typically perform tasks (or compulsion) to seek relief from obsession-related anxiety. Repetitive behaviors such as handwashing, counting, checking, or cleaning are often performed with the hope of preventing obsessive thoughts or making them go away. Performing these "rituals," however, only provides temporary relief. People with OCD may also be diagnosed with a spectrum of other mental disorders, such as generalized anxiety disorder, anorexia nervosa, panic attack, or schizophrenia.

The dysfunction in neuronal communication is considered one of the underlying causes for obsession disorder (Myrrhe van Spronsen and Casper C. Hoogenraad, Curr. Neurol. Neurosci. Rep. 2010, 10, 207-214). Studies suggest that OCD may be related to abnormal levels of a neurotransmitter called serotonin. The first-line treatment of OCD consists of behavioral therapy, cognitive therapy, and medications. Medications for treatment include serotonin reuptake inhibitors (SRIs) such as paroxetine (Seroxat™, Paxil®, Xetanor™, ParoMerck™, Rexetin™), sertraline (Zoloft®, Stimuloton™), fluoxetine (Prozac®, Bioxetin™), escitalopram (Lexapro®), and fluvoxamine (Luvox®) as well as the tricyclic antidepressants, in particular clomipramine (Anafranil®). Benzodiazepines are also used in treatment. As much as 40 to 60% of the patients, however, fail to adequately respond to the SRI therapy and an even greater proportion of patients fail to experience complete remission of their symptoms.

This invention provides methods for treating OCD using the extended release levetiracetam composition of the invention. In certain embodiments, treatment comprises preventing or slowing the progression of OCD. In certain embodiments, treatment comprises alleviation, amelioration, or slowing the progression of one or more symptoms associated with OCD. In certain embodiments, the symptom to be treated is cognitive deficit. For example, methods and compositions of the disclosure can be used to treat the cognitive deficits in OCD, and/or to improve cognitive function in patients with OCD. A quinpirole-sensitized rat model has been developed for OCD. The compulsive checking behavior of the quinpirole-sensitized rats is subject to interruption, which is an attribute characteristic of OCD compulsions. The efficacy of the methods and compositions of this invention in treating OCD, or cognitive deficits associated with OCD, may be assessed in this rat model and other animal models developed for OCD, as well as human subjects with OCD, using a variety of cognitive tests known in the art, as discussed above.

### Substance addiction

Substance addiction (*e.g*., drug substance addiction, alcohol substance addiction) is a mental disorder. The substance addiction is not triggered instantaneously upon exposure to substnace of abuse. Rather, it involves multiple, complex neural adaptations that develop with different time courses ranging from hours to days to months (Kauer J. A. Nat. Rev. Neurosci. 2007, 8, 844-858). The path to substance addiction generally begins with the voluntary use of one or more controlled substances, such as narcotics, barbiturates, methamphetamines, alcohol, nicotine, and any of a variety of other such controlled substances. Over time, with extended use of the controlled substance(s), the voluntary ability to abstain from the controlled substance(s) is compromised due to the effects of prolonged use on brain function, and thus on behavior. As such, substance addiction generally is characterized by compulsive substance craving, seeking and use that persist even in the face of negative consequences. The cravings may represent changes in the underlying neurobiology of the patient which likely must be addressed in a meaningful way if recovery is to be obtained. Substance addiction is also characterized in many cases by withdrawal symptoms, which for some substances are life threatening (*e.g*., alcohol, barbiturates) and in others can result in substantial morbidity (which may include nausea, vomiting, fever, dizziness, and profuse sweating), distress, and decreased ability to obtain recovery. For example, alcoholism, also known as alcohol dependence, is one such substance addiction. Alcoholism is primarily characterized by four symptoms, which include cravings, loss of control, physical dependence and tolerance. These symptoms also may characterize substance addictions to other controlled substances. The craving for alcohol, as well as other controlled substances, often is as strong as the need for food or water. Thus, an alcoholic may continue to drink despite serious family, health and/or legal ramifications.

Recent work exploring the effects of abusing alcohol, central stimulants, and opiates on the central nervous system (CNS) have demonstrated a variety of adverse effects related to mental health, including substance-induced impairments in cognition. See, Nyberg F. *Cognitive Impairments in Drug Addicts,* Chapter 9. In several laboratories and clinics substantial damages of brain function are seen to result from these drugs. Among the harmful effects of the abusing drugs on brain are those contributing to accelerated obsolescence. An observation that has received special attention during recent years is that chronic drug users display pronounced impairment in brain areas associated with executive and memory function. A remarked neuroadaptation caused by addictive drugs, such as alcohol, central stimulants and opiates involves diminished neurogenesis in the subgranular zone (SGZ) of the hippocampus. Indeed, it has been proposed that decreased adult neurogenesis in the SGZ could modify the hippocampal function in such a way that it contributes to relapse and a maintained addictive behavior. It also raises the possibility that decreased neurogenesis may contribute to cognitive deficits elicited by these abusing drugs.

This invention provides methods for treating substance addiction using the extended release levetiracetam composition of the invention. In certain embodiments, treatment comprises preventing or slowing the progression of substance addiction. In certain embodiments, treatment comprises alleviation, amelioration, or slowing the progression of one or more symptoms associated with substance addiction. In certain embodiments, the symptom to be treated is cognitive impairment. For example, methods and compositions of the disclosure can be used to treat the cognitive impairment and/or to improve cognitive function in patients with substance addiction.

Several animal models have been developed to study substance addiction. For example, a genetically selected Marchigian Sardinian alcohol-preferring (msP) rat models is developed to study the neurobiology of alcoholism. See, Ciccocioppo R. et al. Substance addiction Biology 2006, 11, 339-355. The efficacy of the methods and compositions of this invention in treating substance addiction, or cognitive impairment associated with substance addiction, may also be assessed in animal models of substance addiction, as well as human subjects with substance addiction, using a variety of cognitive tests known in the art, as discussed above.

Appropriate methods of administering the extended release compositions of the invention will also depend, for example, on the age of the subject, whether the subject is active or inactive at the time of administering, whether the subject is cognitively impaired at the time of administering, or the extent of the impairment. In some embodiments, the extended release levetiracetam composition of the invention is administered orally, *e.g.,* to a subject by ingestion. In some embodiments, the orally administered composition is administered using a device for extended release.

It will be understood by one of ordinary skill in the art that the compositions and methods described herein may be adapted and modified as is appropriate for the application being addressed and that the compositions and methods described herein may be employed in other suitable applications, and that such other additions and modifications will not depart from the scope hereof.

This invention will be better understood from the Experimental Details which follow. However, one skilled in the art will readily appreciate that the specific methods and results discussed are merely illustrative of the invention as described more fully in the embodiments which follow thereafter.

### Examples

### Example 1: A process for making extended release compositions comprising 190 mg of levetiracetam

**Table 1**

| **Ingredient** | **Functionality** | **Tablet A (M/Tablet)** | **Tablet B (Mg/Tablet)** | **Tablet C (M/Tablet)** |
|---|---|---|---|---|
| Levetiracetam Base | API | 190.0 | 190.0 | 190.0 |
| Hypromellose (Methocel™ K15M CR) | Matrix Former | 300.0 | - | - |
| Hypromellose (Methocel™ K100M Premium CR) | Matrix Former | - | 300.0 | 300.0 |
| Colloidal Silicon Dioxide | Glidant | 1.2 | 1.2 | 1.2 |
| Silicified Microcrystalline Cellulose ProSolv™ HD90 | Diluent | 102.8 | 102.8 | - |
| Encompress, Anhydrous dicalcium phosphate | Diluent | - | - | 102.8 |
| Magnesium Stearate | Lubricant | 6.0 | 6.0 | 6.0 |
| Total | | 600 | 600 | 600 |

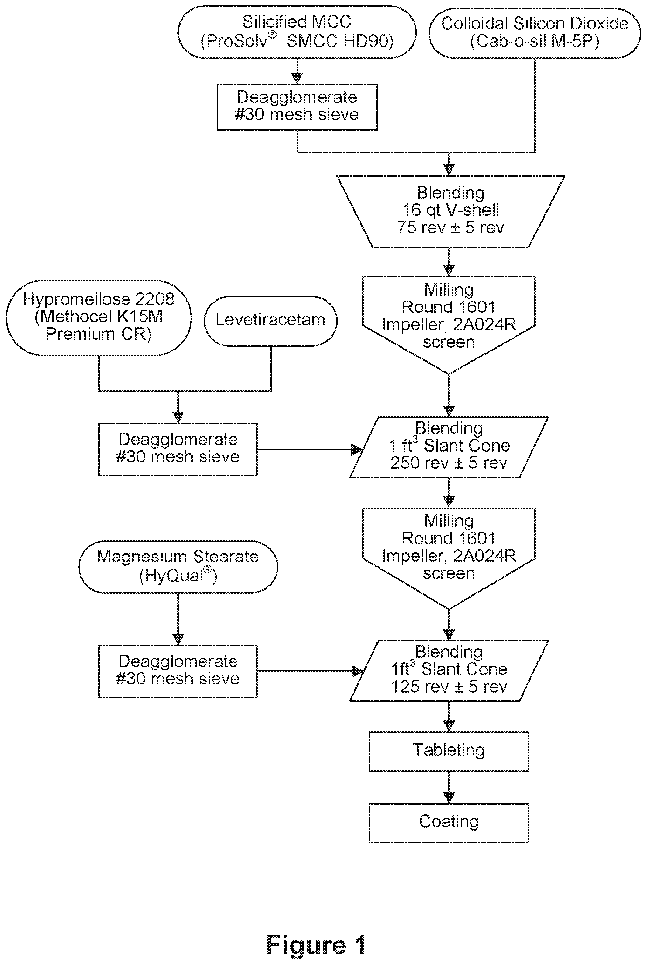

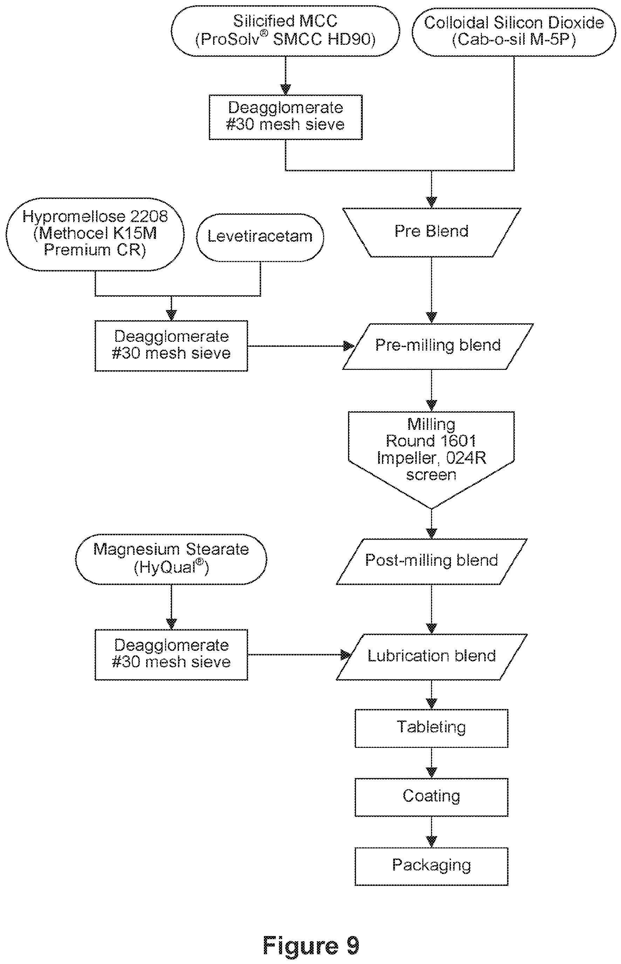

Three extended release tablets A, B, and C comprising 190 mg of levetiracetam as shown in **Table 1** are manufactured following the process exemplified in the flow diagram of **Figure 1****.** The process exemplified in the flow diagram of **Figure 9** could also be used. In brief, Silicified Microcrystalline Cellulose ProSolv™ SMCC HD90 (or Encompress, Anhydrous dicalcium phosphate) is sifted through deagglomerate #30 U.S. mesh sieve, and then blended with Colloidal Silicon Dioxide (16 qt V-shell blender; 75 rev ± 5 rev). The blended sample then goes through Round 1601 Impeller (2A024R screen). 190 mg of levetiracetam and hypromellose 2208 (Methocel™ K15M Premium CR) (or Methocel™ K100M Premium CR) are also sifted through deagglomerate #30 U.S. mesh sieve, and then blended in a 1ft³ Slant Cone Blender (250 rev ± 5 rev) with the ground Silicified Microcrystalline Cellulose ProSolv™ HD90 and Colloidal Silicon Dioxide. This blended sample then goes through Round 1601 Impeller (2A024R screen) and then is blended in a 1ft³ Slant Cone Blender (125 rev ± 5 rev) with sieved Magnesium Stearate (HyQual®) (sieved through deagglomerate #30 U.S. mesh sieve). The blended samples are compressed into tablets. Optionally, the tablets are further film coated with a hypromellose-based (HPMC-based) coating, such as Opadry® complete film coating system.

### Example 2: Dissolution profile of extended release compositions comprising 190 mg of levetiracetam

**Table 2** below shows the dissolution profile for the 190 mg levetiracetam extended release Tablet A of **Table 1.**

**Table 2**

| **Test** | **Results (Time: Percentages of dissolution)** |
|---|---|
| Dissolution | 1 hr: 29% |
| | 3 hr: 51% |
| | 12 hr: 92% |

When extended release Tablet A is placed in variety of EtOH concentrations in 0.1N HCL, no dose dumping is observed.

### Example 3: A process for making extended release compositions comprising 220 mg of levetiracetam

**Table 3**

| **Ingredient** | **Functionality** | **Tablet D (Mg/Tablet)** | **Tablet E (Mg/Tablet)** |
|---|---|---|---|
| Levetiracetam | API | 220.0 | 220.0 |
| Hypromellose (Methocel™ K15M CR) | Matrix Former | 280.0 | 347.5 |
| Colloidal Silicon Dioxide | Glidant | 1.2 | 1.4 |
| Silicified Microcrystalline Cellulose ProSolv™ HD90 | Diluent | 92.8 | 119.2 |
| Magnesium Stearate | Lubricant | 6.0 | 6.7 |
| Total | | 600 | 695 |

Two extended release tablets D and E comprising 220 mg of levetiracetam as shown in **Table 3** are manufactured following the process exemplified in the flow diagram of **Figure 1****.** The process exemplified in the flow diagram of **Figure 9** could also be used. In brief, Silicified Microcrystalline Cellulose ProSolv™ SMCC HD90 (or Encompress, Anhydrous dicalcium phosphate) is sifted through deagglomerate #30 U.S. mesh sieve, and then blended with Colloidal Silicon Dioxide (16 qt V-shell blender; 75 rev ± 5 rev). The blended sample then goes through Round 1601 Impeller (2A024R screen). 220 mg of levetiracetam and hypromellose 2208 (Methocel™ K15M Premium CR) (or Methocel™ K100M Premium CR) are also sifted through deagglomerate #30 U.S. mesh sieve, and then blended in a 1ft³ Slant Cone Blender (250 rev ± 5 rev) with the ground Silicified Microcrystalline Cellulose ProSolv™ HD90 and Colloidal Silicon Dioxide. This blended sample then goes through Round 1601 Impeller (2A024R screen) and then is blended in a 1ft³ Slant Cone Blender (125 rev ± 5 rev) with sieved Magnesium Stearate (HyQual®) (sieved through deagglomerate #30 U.S. mesh sieve). The blended samples are compressed into tablets. Optionally, the tablets are further film coated with a hypromellose-based (HPMC-based) coating, such as Opadry® complete film coating system.

### Example 4: Dissolution Profile of Extended Release Compositions Comprising 220 mg Of Levetiracetam

**Table 4** below shows the dissolution profile for the 220 mg levetiracetam extended release Tablet D of **Table 3.**

**Table 4**

| **Test** | **Results (Time: Percentages of dissolution)** |
|---|---|
| Dissolution | 1 hr: 28% |
| | 3 hr: 49% |
| | 12 hr: 91% |

When extended release Tablet D is placed in variety of EtOH concentrations in 0.1N HCL, no dose dumping is observed.

### Example 5: Evaluation of extended release compositions of 190 mg levetiracetam on pharmacokinetics in dogs

### Overview

The purpose of this study is to collect samples for investigating the pharmacokinetics of novel extended release formulations of levetiracetam (190 mg) in male dogs following oral administration. **Table 1** provides a description of the three formulations utilized in this study (190 mg Tablets A, B, and C).

### Animals

Thirty non-naive male purebred beagle dogs from the Covance Stock colony are used in these studies. The animals are acclimated to study conditions for approximately three days prior to dose administration. At dosing, the animals weigh 8.4 to 12.8 kg and are 1 to 2 years of age. All animals are housed in individual, stainless steel cages during acclimation and the test period, except during periods of commingling in accordance with Covance SOPs. Certified Harlan Teklad 2021, 21% Protein Dog Diet is provided ad libitum unless otherwise specified for dose administration. Water is provided fresh daily, ad libitum. Environmental controls for the animal room are set to maintain a temperature of 68 to 79°F, a relative humidity of 50 ± 20%, and a 12-hour light/12-hour dark cycle. As necessary, the 12-hour dark cycle is interrupted to accommodate study procedures.

### Study Design

Five groups of dogs (N=6 per group) are utilized in this study. An immediate release 250 mg levetiracetam (LEV IR) tablet is administered as a 250 mg oral BID regimen (a total dose of 500). An extended release 500 mg levetiracetam tablet (LEV XR) is administered as a single oral dose of 500 mg. Tablets A, B, and C are administered as single oral doses of 190 mg. Plasma pharmacokinetic samples are collected at pre-dose, 0.25, 0.5, 1, 2, 4, 6, 8, 12, 13 (collecting only LEV IR), 18, 24, and 48 hours post dose. For LEV IR, the 12-hour blood sample is collected just prior to administration of the second dose.

**Table 5: Overview of Study Design**

| Group | Number of Male Animals | Test Article | Dose Route | Target Dose Level (mg/tablet) |
|---|---|---|---|---|
| 1 | 6 | LEV - IR | Oral Tablet | 250 |
| 2 | 6 | LEV - XR | Oral Tablet | 500 |
| 3 | 6 | Tablet A | Oral Tablet | 190 |
| 4 | 6 | Tablet B | Oral Tablet | 190 |
| 5 | 6 | Tablet C | Oral Tablet | 190 |

| | | | | |
|---|---|---|---|---|
| IR Immediate release. XR Extended release. Notes: Animals in Group 1 receive two 250 mg tablets, approximately 12 hours apart (500 mg total dose). Animals in Groups 2 through 5 receive a single tablet. | | | | |

### Analytical Methods

Sample analysis is performed using a Covance-owned generic method and analog internal standard. The method is adjusted as appropriate for the specific test article used on study. Data collection and chromatographic interpretation is performed in Analyst and the Laboratory Information Management System (LIMS) used on study is Watson.

### Absorption and Plasma Levels

LEV IR vs. LEV XR: LEV IR is administered as a 250 mg oral BID regimen (total daily dose of 500 mg). LEV XR is administered as a single oral dose of 500 mg. Based on mean plasma Cₘₐₓ and AUC_{0-inf}, the overall exposure of dogs to levetiracetam is similar with both formulations **(****Figure 2****, Table 6).** The plasma Tₘₐₓ is earlier with the IR formulation (range 0.25 - 2.00 h; mean 1.00 h) than with the XR formulation (range 2.00 - 4.00 h; mean 3.33 h). The apparent elimination half-life of levetiracetam in plasma averages 3.50 ± 0.273 h and 4.23 ± 0.590 h with the LEV IR and LEV XR formulations, respectively.

190 mg Tablets A, B, and C: 190 mg Tablets A, B, and C of **Table 1** are administered as single oral doses of 190 mg. The highest exposure to levetiracetam is achieved with Tablet A **(****Figure 2****, Table 6);** plasma Cₘₐₓ and AUC_{0-inf} averaged 8650 ± 1440 ng/mL and 90000 ± 27200 ng•h/mL, respectively. The plasma Tₘₐₓ generally range from 2.00 to 4.00 hours. The apparent elimination half-life of levetiracetam in plasma average 4.15 ± 1.26h.

**Table 6: Pharmacokinetic parameters in plasma collected from male dogs following oral administration of levetiracetam**

| Animal | | Cₘₐₓ | Cₘₐₓ/D | Tₘₐₓ | AUC₀₋ₜ | AUC_{0-inf} | AUC_{0-inf}/D | t_{1/2} |
|---|---|---|---|---|---|---|---|---|
| Number | Group | (ng/mL) | ((ng/mL)/mg) | (h) | (h·ng/mL) | (h·ng/mL) | ((h•ng/mL)/mg) | (h) |
| 250mg BID(LEV - IR) | | | | | | | | |
| 112515 | 1 | 29200 | 58.4 | 0.50 | 247000 | 247000 | 493 | 3.32 |
| 113624 | 1 | 23200 | 46.4 | 0.25 | 237000 | 237000 | 474 | 3.45 |
| 113626 | 1 | 31400 | 62.8 | 0.25 | 302000 | 302000 | 604 | 3.44 |
| 113648 | 1 | 38100 | 76.2 | 2.00 | 277000 | 277000 | 554 | 3.15 |
| 113627 | 1 | 24300 | 48.6 | 2.00 | 280000 | 280000 | 560 | 3.83 |
| 113614 | 1 | 24500 | 49.0 | 1.00 | 214000 | 214000 | 428 | 3.82 |
| | Mean | 28500 | 56.9 | 1.00 | 259000 | 259000 | 519 | 3.50 |
| | SD | 5710 | 11.4 | 0.822 | 32400 | 32400 | 64.8 | 0.273 |

| 500mg(LEV - XR) | | | | | | | | |
|---|---|---|---|---|---|---|---|---|
| 113637 | 2 | 36900 | 73.8 | 4.00 | 218000 | 218000 | 436 | 3.73 |
| 113643 | 2 | 25000 | 50.0 | 2.00 | 265000 | 265000 | 531 | 5.19 |
| 113646 | 2 | 31600 | 63.2 | 4.00 | 302000 | 302000 | 604 | 4.16 |
| 112513 | 2 | 35400 | 70.8 | 2.00 | 317000 | 317000 | 634 | 3.74 |
| 113638 | 2 | 24900 | 49.8 | 4.00 | 230000 | 230000 | 460 | 4.69 |
| 113615 | 2 | 44300 | 88.6 | 4.00 | 287000 | 287000 | 574 | 3.89 |
| | Mean | 33000 | 66.0 | 3.33 | 270000 | 270000 | 540 | 4.23 |
| | SD | 7490 | 15.0 | 1.03 | 39600 | 39600 | 79.1 | 0.590 |

| 190mg(TabletA) | | | | | | | | |
|---|---|---|---|---|---|---|---|---|
| 112417 | 3 | 9900 | 52.1 | 2.00 | 122000 | 122000 | 644 | 3.51 |
| 113636 | 3 | 10100 | 53.2 | 4.00 | 108000 | 108000 | 571 | 3.94 |
| 113616 | 3 | 7740 | 40.7 | 2.00 | 125000 | NR | NR | NR |
| 113613 | 3 | 7310 | 38.5 | 2.00 | 53100 | 53100 | 280 | 6.36 |
| 113618 | 3 | 7000 | 36.8 | 2.00 | 75600 | 75600 | 398 | 3.23 |
| 112516 | 3 | 9820 | 51.7 | 4.00 | 90600 | 90600 | 477 | 3.69 |
| | Mean | 8650 | 45.5 | 2.67 | 95900 | 90000 | 474 | 4.15 |
| | SD | 1440 | 7.58 | 1.03 | 28200 | 27200 | 143 | 1.26 |

| | | | | | | | | |
|---|---|---|---|---|---|---|---|---|
| **AUC₀₋ₜ Area under the plasma concentration-time curve up to the last sampling time with measurable concentrations.** **AUC_{0-inf} Area under the plasma concentration-time curve up to infinity.** **AUC_{0-inf}/D Dose adjusted area under the plasma concentration-time curve up to infinity.** **Cₘₐₓ Maximum plasma concentration.** **Cₘₐₓ/D Dose adjusted maximum plasma concentration.** **h Hours.** **IR Immediate release.** **NR Not reported.** **SD Standard deviation.** **Tₘₐₓ Time to maximum concentration.** **t_{1/2} Observed elimination half-life.** **XR Extended release.** | | | | | | | | |

The highest overall exposure of levetiracetam in dogs is achieved with the LEV XR and LEV IR formulations. Of the 190 mg Tablets A, B, and C, the highest overall exposure of levetiracetam in dogs is achieved with the 190 mg Tablet A. Based on the mean dose-adjusted plasma Cₘₐₓ and AUC_{0-inf} values, the levetiracetam exposure achieved with the 190 mg Tablet A is approximately 69% and 88%, respectively, of the exposure achieved with the LEV XR formulation, and 80% and 91%, respectively, of the exposure achieved with the LEV IR formulation.

### Example 6: Evaluation of extended release compositions of 220 mg levetiracetam on pharmacokinetics in dogs

### Overview

The purpose of this study is to collect samples for investigating the pharmacokinetics of novel extended release formulations of levetiracetam (220 mg) in male dogs following oral administration. **Table 3** provides a description of the two formulations utilized in this study (220 mg Tablets D and E).

### Animals

Eighteen non-naive male purebred beagle dogs from the Covance Stock colony are used in these studies. The animals are acclimated to study conditions for approximately three days prior to dose administration. At dosing, the animals weigh 7.6 to 11.7 kg and are approximately 1 year of age. All animals are housed in individual, stainless steel cages during acclimation and the test period, except during periods of commingling in accordance with Covance SOPs. Certified Harlan Teklad 2021, 21% Protein Dog Diet is provided *ad libitum* unless otherwise specified for dose administration. Water is provided fresh daily, *ad libitum.* Environmental controls for the animal room are set to maintain a temperature of 68 to 79°F, a relative humidity of 50 ± 20%, and a 12-hour light/12-hour dark cycle. As necessary, the 12-hour dark cycle is interrupted to accommodate study procedures.

### Study Design

Three groups of dogs (N=6 per group) are utilized in this study. LEV XR is administered as a single oral dose of 500 mg. 220 mg Tablets D and E are administered as single oral doses of 220 mg. Plasma pharmacokinetic samples are collected at pre-dose (*i.e.,* 0), 0.25, 0.5, 1, 2, 4, 6, 8, 12, 18, 24, and 48 hours post dose. See **Table 7.**

**Table 7: Overview of Study Design**

| Group | Number of Male Animals | Test Article | Dose Route | Target Dose Level (mg/tablet) |
|---|---|---|---|---|
| 1 | 6 | LEV-XR | Oral Tablet | 500 |
| 2 | 6 | Tablet D | Oral Tablet | 220 |
| 3 | 6 | Tablet E | Oral Tablet | 220 |

| | | | | |
|---|---|---|---|---|
| XR Extended Release. Note: Animals received a single tablet. | | | | |

### Analytical Methods

Sample analysis is performed using a Covance-owned generic method and analog internal standard. The method is adjusted as appropriate for the specific test article used on study. Data collection and chromatographic interpretation are performed in Analyst and the Laboratory Information Management System (LIMS) used on study is Watson.

### Absorption and Plasma Levels

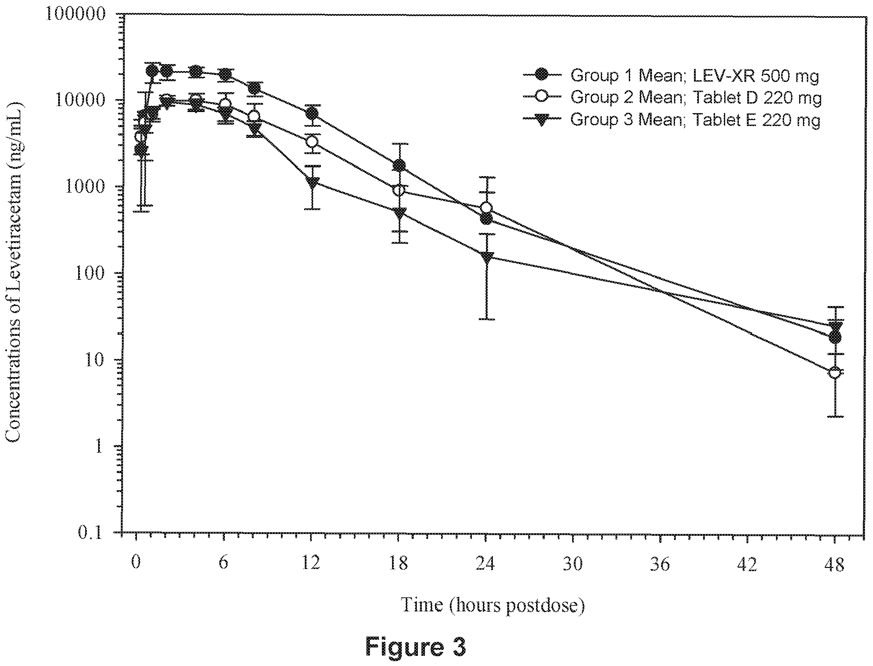

LEV-XR is administered as a single oral tablet dose of 500 mg; the 220 mg Tablets D and E are each administered as single oral tablet doses of 220 mg. Based on mean dose-adjusted plasma Cₘₐₓ and AUC₀₋ₜ, the overall exposure of dogs to levetiracetam is similar with the LEV-XR and the 220 mg Tablet D formulations (**Figure 3****, Table 8**).

### LEV XR vs 220 mg Tablets

The plasma Cₘₐₓ and AUC_{0-inf} for the 220 mg Tablet D average 10900± 2540 ng/mL and 110000 ± 23000 ng•h/mL, respectively. The plasma Tₘₐₓ generally ranges from 2.00 to 6.00 hours. The apparent elimination half-life of levetiracetam in plasma averages 4.41 ± 0.06 h. The mean dose-adjusted plasma Cₘₐₓ values are 46.6 ± 7.37 and 49.3 ± 11.5 ng/mL for the LEV-XR and the 220 mg Tablet D respectively. The dose-adjusted plasma AUC₀₋ₜ values are 452 ± 67.2 and 499 ± 104 ng•h/mL for the LEV-XR and the 220 mg Tablet D, respectively.

The plasma Tₘₐₓ is similar for LEV XR and the 220 mg Tablet D (LEV-XR: range 1.0 - 3.6 h; mean 1.6 h; the 220 mg Tablet D: range 2.0-6.0 h; mean 2.7 h). The apparent elimination half-life of levetiracetam in plasma averages 5.16 ± 1.44 h and 4.41 ± 0.614 h with the LEV-XR and the 220 mg Tablet D formulations, respectively.

**Table 8. Pharmacokinetic parameters in plasma collected from male dogs following oral administration of Levetiracetam**

| **Group** | **Dose (mg)** | **Subject No.** | **Cₘₐₓ (ng/mL)** | **Cₘₐₓ/D (ng/mL)/mg** | **Tₘₐₓ (h)** | **AUC₀₋ₜ, (ng•h/mL)** | **AUC₀₋ₜ / D (ng•h/mL)/mg** | **AUC_{0-inf} (ng•h/mL)** | **t_{1/2} (h)** |
|---|---|---|---|---|---|---|---|---|---|
| 500mg(LEV - XR) | | | | | | | | | |
| **1** | 500 | 113637 | 25600 | 51.2 | 1.0 | 259000 | 518 | 259000 | 3.75 |
| **1** | 500 | 113638 | 23700 | 47.4 | 3.6 | 201000 | 402 | 201000 | 7.17 |
| **1** | 500 | 113639 | 28800 | 57.6 | 1.0 | 263000 | 526 | 263000 | 5.21 |
| **1** | 500 | 113641 | 19500 | 39.0 | 1.0 | 192000 | 384 | 192000 | 6.35 |
| **1** | 500 | 113642 | 19100 | 38.2 | 2.0 | 195000 | 390 | 196000 | 5.01 |
| **1** | 500 | 113647 | 23100 | 46.2 | 1.0 | 247000 | 494 | 247000 | 3.44 |
| | | N | 6 | 6 | 6 | 6 | 6 | 6 | 6 |
| | | Mean | 23300 | 46.6 | 1.6 | 226000 | 452 | 226000 | 5.16 |
| | | SD | 3680 | 7.37 | 1.1 | 33500 | 67.2 | 33400 | 1.44 |
| | | CV% | 15.8 | 15.8 | 66.1 | 14.8 | 14.9 | 14.8 | 28.0 |

| 220mg(TabletD) | | | | | | | | | |
|---|---|---|---|---|---|---|---|---|---|
| **2** | 220 | 113700 | 15300 | 69.5 | 6.0 | 142000 | 645 | 142000 | 4.65 |
| **2** | 220 | 113814 | 10400 | 47.3 | 2.0 | 108000 | 491 | 108000 | 3.91 |
| **2** | 220 | 113817 | 11400 | 51.8 | 2.0 | 130000 | 591 | NR | NR |
| **2** | 220 | 113832 | 10000 | 45.5 | 2.0 | 104000 | 473 | 104000 | 3.74 |
| **2** | 220 | 114110 | 10500 | 47.7 | 2.0 | 96100 | 437 | 96200 | 5.27 |
| **2** | 220 | 114111 | 7520 | 34.2 | 2.0 | 79000 | 359 | 79000 | 4.50 |
| | | N | 6 | 6 | 6 | 6 | 6 | 5 | 5 |
| | | Mean | 10900 | 49.3 | 2.7 | 110000 | 499 | 106000 | 4.41 |
| | | SD | 2540 | 11.5 | 1.6 | 23000 | 104 | 23200 | 0.614 |
| | | CV% | 23.4 | 23.4 | 61.2 | 20.9 | 20.8 | 21.9 | 13.9 |

| | | | | | | | | | |
|---|---|---|---|---|---|---|---|---|---|
| AUC₀₋ₜ Area under the plasma concentration-time curve up to the last sampling time with measurable concentrations. AUC_{0-inf} Area under the plasma concentration-time curve up to infinity. AUC_{0-inf}/D Dose adjusted area under the plasma concentration-time curve up to infinity. Cₘₐₓ Maximum plasma concentration. Cₘₐₓ/D Dose adjusted maximum plasma concentration. CV% Coefficient of variation. h Hours. N Number of animals. NR Not reported due to ill-defined terminal phase. SD Standard deviation. Tₘₐₓ Time to maximum concentration. t_{1/2} Observed elimination half-life. | | | | | | | | | |

Based on the mean dose-adjusted plasma Cₘₐₓ and AUC₀₋ₜ values, the levetiracetam exposure achieved with the 220 mg Tablet D formulation is approximately 107% and 110%, respectively, of the exposure achieved with the LEV-XR formulation.

### Example 7: Phase I food effect study of extended release compositions of 190 mg and 220 mg levetiracetam

This example describes a two-group, single-dose, two-period, two-way crossover, food-effect study of two extended release levetiracetam formulations, *i.e.,* the 190 mg Tablet A of **Table 1** and the 220 mg Tablet D of **Table 3.**

### Objective

The objective of this study is to assess the effect of food on the rate and extent of absorption of two extended release levetiracetam formulations, *i.e*., the 190 mg Tablet A of **Table 1** and the 220 mg Tablet D of **Table 3.**

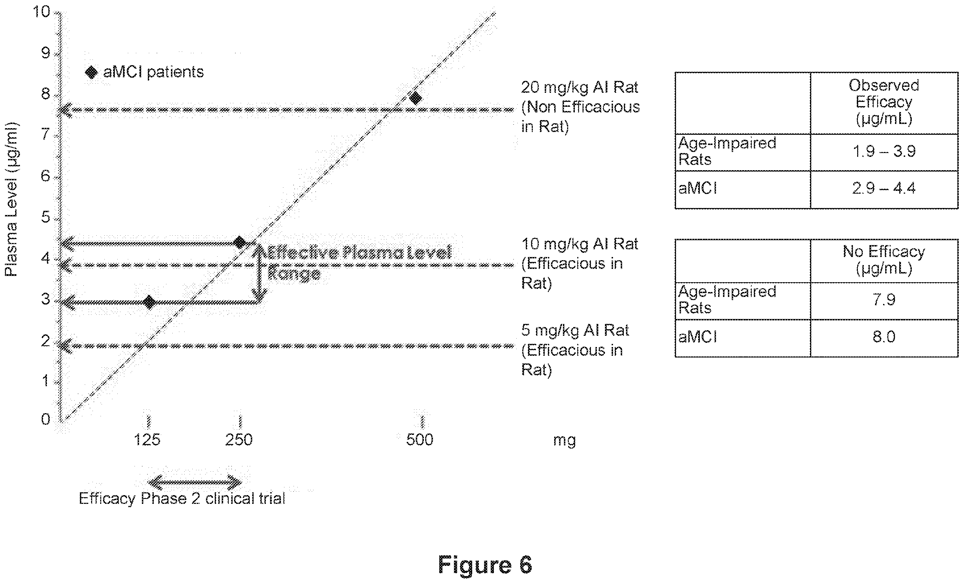

Steady state formulation goals: The preferred range goal is established based on aMCI phase II human study: between 2.9 and 4.4 µg/mL. The acceptable range goal is established based on Aged-Impaired (AI) rats and aMCI phase II human study: between 1.9 and 4.4 µg/mL. See **Figure 6****.**

### Study Design

This is an open label, randomized, two-group, single-dose, two-period crossover, food-effect study. Fifty-six (56) healthy subjects are enrolled. Subjects who successfully complete the screening process check into the research center the evening before first dose. Subjects who continue to meet inclusion/exclusion criteria the morning of dose are assigned a subject number, based on the order in which they successfully complete the required screening process and procedures. Dosing days are separated by a washout period of at least 7 days.

Subjects are randomly assigned to one of two groups:

**Group 1: Subjects (n = 28) received extended-release Tablet A of Table 1 (190 mg).**

| | |
|---|---|
| Treatment A: | Tablet A |
| | Dose = 1 x 190 mg tablet, orally administered under fasted conditions |
| Treatment B: | Tablet A |
| | Dose = 1 x 190 mg tablet, orally administered under fed conditions |

**Group 2: Subjects (n = 28) received extended-release Tablet D of Table 3 (220 mg).**

| | |
|---|---|
| Treatment A: | Tablet D |
| | Dose = 1 x 220 mg tablet, orally administered under fasted conditions |
| Treatment B: | Tablet D |
| | Dose = 1 x 220 mg tablet, orally administered under fed conditions |

Fed treatment: Following an overnight fast of at least 10 hours, subjects began consuming a Food and Drug Administration (FDA) standard high-calorie, high-fat breakfast meal 30 minutes prior to administration of the study drug.

Fasted treatment: Subjects are dosed after an overnight fast of at least 10 hours.

Each drug administration is separated by a washout period of at least 7 days.

Each dose is orally administered along with approximately 240 mL (8 fl. oz.) of room temperature water. After dosing, no food is allowed until 4 hours postdose. Except for the 240 mL of room temperature water provided with the dose, no water might be consumed for 1 hour prior through 1 hour after dose. Water consumption followed the guidelines in Section 5.4.2. With the exception of the FDA standard high-calorie, high-fat breakfast meal served during the fed treatment period, meals are the same and scheduled at approximately the same times relative to dose for each study period.

Subjects who withdraw from the study are not replaced.

### Clinical Procedures Summary

During each study period, 6 mL blood samples are obtained prior to each dosing and following each dose at selected times through 24 hours post-dose. A total of 34 pharmacokinetic blood samples are to be collected from each subject, 17 samples in each study period. In addition, blood is drawn and urine is collected for clinical laboratory testing at screening and study exit.

In each study period, subjects are admitted to the study unit in the evening prior to the scheduled dose. Subjects are confined to the research center during each study period until completion of the 24-hour blood collection and other study procedures.

### Procedures for Collecting Samples for Pharmacokinetic Analysis

Blood samples (1 x 6 mL) are collected in vacutainer tubes containing K₂-EDTA as a preservative at pre-dose (0) and at 1.0, 2.0, 3.0, 4.0, 4.5, 5.0, 5.5, 6.0, 6.5, 7.0, 8.0, 9.0, 10, 12, 18, and 24 hours after dosing.

### Bioanalytical Summary

Plasma samples are analyzed for levetiracetam using a validated LC-MS-MS procedure. The method is validated for a range of 0.0500 to 30.0 µg/mL for levetiracetam, based on the analysis of 0.200 mL of human EDTA plasma. Data are stored in Watson Laboratory Information Management System (LIMS; Version 7.2.0.03, Thermo Fisher Scientific).

### Pharmacokinetic Analysis

Data are analyzed by noncompartmental methods in WinNonlin. Concentration-time data that are below the limit of quantification (BLQ) are treated as zero in the data summarization and descriptive statistics. In the pharmacokinetic analysis, BLQ concentrations are treated as zero from time-zero up to the time at which the first quantifiable concentration is observed; embedded and/or terminal BLQ concentrations are treated as "missing". Actual sample times are used for all pharmacokinetic and statistical analyses.

The following pharmacokinetic parameters are calculated: peak concentration in plasma (Cₘₐₓ), time to peak concentration (Tₘₐₓ), elimination rate constant (λ_{z}), terminal half-life (T_{1/2}), area under the concentration-time curve from time-zero to the time of the last quantifiable concentration (AUCₗₐₛₜ), and area under the plasma concentration time curve from time-zero extrapolated to infinity (AUC_{inf}). Additionally, Cₘₐₓ, AUCₗₐₛₜ, and AUC_{inf} are dose-normalized.

Analysis of variance (ANOVA) and the Schuirmann's two one-sided t-test procedures at the 5% significance level are applied to the log-transformed pharmacokinetic exposure parameters, Cₘₐₓ, AUCₗₐₛₜ, and AUC_{inf}. The 90% confidence interval for the ratio of the geometric means (Test/Reference) is calculated. A lack of food effect is declared if the lower and upper confidence intervals of the log-transformed parameters are within 80% to 125% (190 mg Tablet A Fed vs. 190 mg Tablet A Fasted; 220 mg Tablet D Fed vs. 220 mg Tablet D Fasted). Additionally, the dose-normalized Cₘₐₓ, AUCₗₐₛₜ, and AUC_{inf} are compared within fasted and fed conditions to determine dose-proportionality. Dose-proportionality is concluded if the lower and upper confidence intervals of the dose-normalized, log-transformed parameters are within 80% to 125% (220 mg Tablet D Fasted vs. 190 mg Tablet A Fasted; 220 mg Tablet D Fed vs. 190 mg Tablet A Fed)

### Results

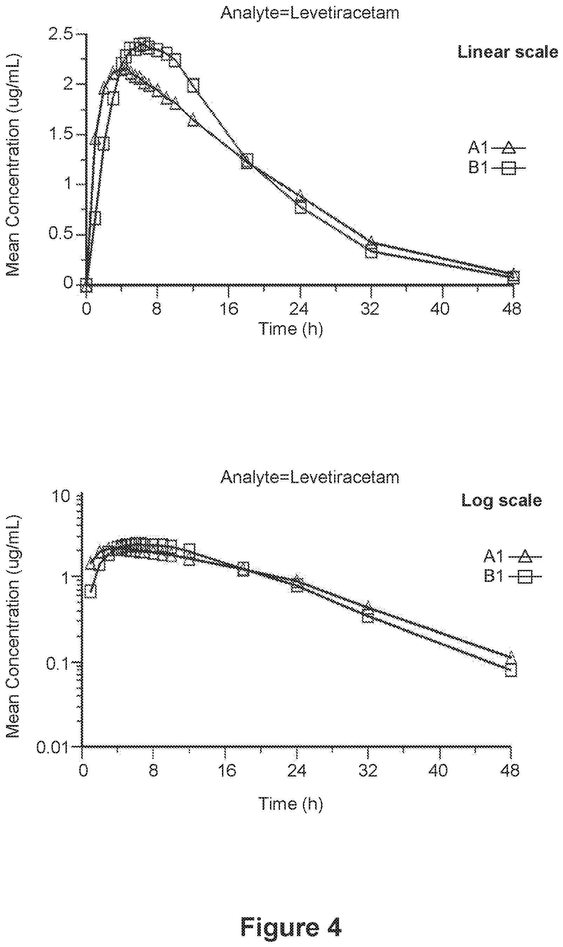

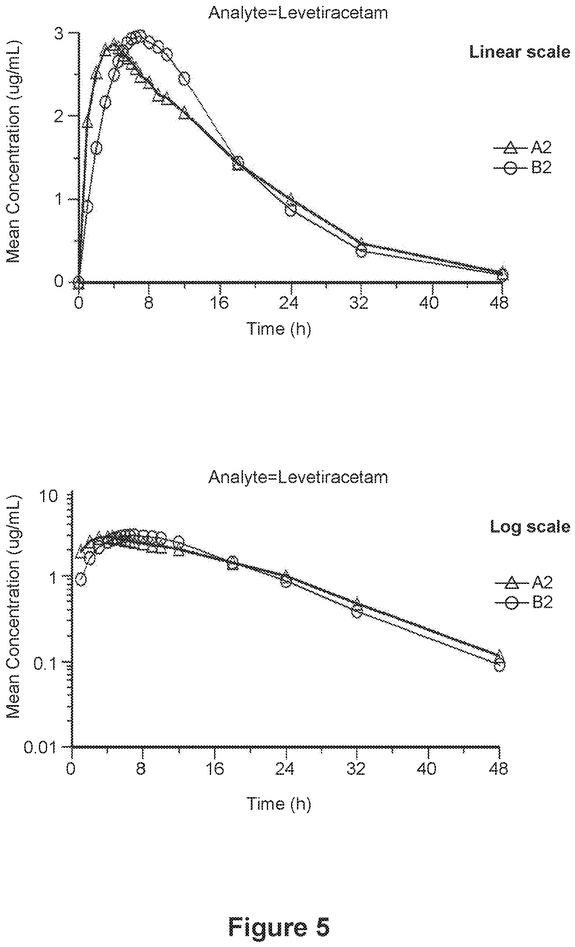

Data from 55 subjects for Group 1 and 54 subjects for Group 2 are included in the pharmacokinetic and statistical analyses. Mean concentration-time data are shown in **Tables 9 and 10** and in **Figures 4** **and** **5****.** Results of the pharmacokinetic and statistical analyses are shown below in **Tables 9-15.**

### Conclusions

### Food-Effect:

The 90% confidence intervals for the log-transformed exposure parameters Cₘₐₓ, AUCₗₐₛₜ, and AUC_{inf} are within the 80% to 125% range for the 190 mg and 220 mg doses. The presence of food does not alter the pharmacokinetics of the 190 mg and 220 mg levetiracetam doses.

### Dose Proportionality:

The 90% confidence intervals for the dose-normalized log-transformed exposure parameters Cₘₐₓ/D, AUCₗₐₛₜ/D, and AUC_{inf}/D are within the 80% to 125% range for fed and fasted conditions. Levetiracetam exposure, as measured by Cₘₐₓ/D, AUCₗₐₛₜ/D, and AUC_{inf}/D, increase proportionately from 190 mg (Tablet A) to 220 mg (Tablet D).

### Steady State Modeling

According to the steady state modeling of PK profile for the 190 mg Tablet A, it meets the acceptable range goal, *i.e.,* between 1.9 and 4.4 µg/ml. See

### Figure 7.

According to the steady state modeling of PK profile for the 220 mg Tablet D, it meets the preferred range goal, *i.e.,* between 2.9 and 4.4 µg/ml. See

### Figure 8.

**Table 9: Levetiracetam Concentration-Time Data after Administration of Extended-Release Tablet A, 190 mg under Fasted Conditions (Group 1/Treatment A) and Extended-Release Tablet A, 190 mg under Fed Conditions (Group 1/Treatment B)**

| | **Group 1/TreatmentA:** | | | | **Group 1/TreatmentB:** | | | |
|---|---|---|---|---|---|---|---|---|
| | **190mg Tablet A, Fasted** | | | | **190mg Tablet A, Fed** | | | |
| **Time (h)** | **n** | **Mean (µg/mL)** | **SD (µg/mL)** | **CV (%)** | **n** | **Mean (µg/mL)** | **SD (µg/mL)** | **CV (%)** |
| 0.00 | 28 | 0.00 | 0.00 | NC | 27 | 0.00 | 0.00 | NC |
| 1.00 | 28 | 1.46 | 0.450 | 30.76 | 27 | 0.665 | 0.351 | 52.82 |
| 2.00 | 28 | 1.96 | 0.535 | 27.21 | 27 | 1.41 | 0.454 | 32.20 |
| 3.00 | 28 | 2.11 | 0.548 | 25.93 | 27 | 1.86 | 0.391 | 21.03 |
| 4.00 | 28 | 2.15 | 0.569 | 26.53 | 27 | 2.19 | 0.443 | 20.25 |
| 4.50 | 28 | 2.16 | 0.549 | 25.42 | 27 | 2.27 | 0.469 | 20.70 |
| 5.00 | 28 | 2.11 | 0.508 | 24.08 | 27 | 2.34 | 0.500 | 21.35 |
| 5.50 | 28 | 2.08 | 0.497 | 23.91 | 27 | 2.34 | 0.519 | 22.15 |
| 6.00 | 28 | 2.06 | 0.445 | 21.61 | 27 | 2.38 | 0.514 | 21.58 |
| 6.50 | 28 | 2.02 | 0.445 | 22.07 | 27 | 2.39 | 0.516 | 21.57 |
| 7.00 | 28 | 1.99 | 0.437 | 21.97 | 27 | 2.36 | 0.472 | 20.04 |
| 8.00 | 28 | 1.94 | 0.451 | 23.20 | 27 | 2.34 | 0.517 | 22.13 |
| 9.00 | 28 | 1.86 | 0.440 | 23.64 | 27 | 2.30 | 0.543 | 23.64 |
| 10.00 | 28 | 1.81 | 0.464 | 25.58 | 27 | 2.24 | 0.568 | 25.41 |
| 12.00 | 28 | 1.65 | 0.402 | 24.42 | 27 | 1.98 | 0.471 | 23.74 |
| 18.00 | 28 | 1.23 | 0.339 | 27.62 | 27 | 1.24 | 0.306 | 24.63 |
| 24.00 | 28 | 0.888 | 0.272 | 30.68 | 27 | 0.783 | 0.212 | 27.07 |
| 32.00 | 28 | 0.431 | 0.140 | 32.52 | 27 | 0.342 | 0.0991 | 29.00 |
| 48.00 | 27 | 0.112 | 0.0517 | 46.22 | 27 | 0.0798 | 0.0442 | 55.39 |

| | | | | | | | | |
|---|---|---|---|---|---|---|---|---|
| Note: Plasma samples analyzed using a bioanalytical method with a validated range 0.0500 to 30.0 ug/mL; concentrations reported in µg/mL to 3 significant figures; concentrations below limit of quantification set to zero (0.00 µg/mL) in the data summarization NC = Not calculated | | | | | | | | |

**Table 10: Levetiracetam Concentration-Time Data after Administration of Extended-Release Tablet D, 220 mg under Fasted Conditions (Group 2/Treatment A) and Extended-Release Tablet D, 220 mg under Fed Conditions (Group 2/Treatment B)**

| | **Group 2/TreatmentA**: | | | | **Group 2/Treatment B:** | | | |
|---|---|---|---|---|---|---|---|---|
| | **220 mg Tablet D, Fasted** | | | | **220 mg Tablet D, Fed** | | | |
| **Time (h)** | **n** | **Mean (µg/mL)** | **SD (µg/mL)** | **CV (%)** | **n** | **Mean (µg/mL)** | **SD (µg/mL)** | **CV (%)** |
| 0.00 | 26 | 0.00 | 0.00 | NC | 28 | 0.00 | 0.00 | NC |
| 1.00 | 26 | 1.94 | 0.619 | 31.91 | 28 | 0.911 | 0.681 | 74.77 |
| 2.00 | 26 | 2.53 | 0.645 | 25.53 | 28 | 1.61 | 0.636 | 39.41 |
| 3.00 | 26 | 2.80 | 0.618 | 22.08 | 28 | 2.16 | 0.560 | 25.89 |
| 4.00 | 26 | 2.86 | 0.596 | 20.83 | 28 | 2.49 | 0.558 | 22.36 |
| 4.50 | 26 | 2.83 | 0.553 | 19.57 | 28 | 2.65 | 0.506 | 19.07 |
| 5.00 | 26 | 2.73 | 0.501 | 18.33 | 28 | 2.78 | 0.454 | 16.35 |
| 5.50 | 26 | 2.70 | 0.499 | 18.47 | 28 | 2.87 | 0.474 | 16.49 |
| 6.00 | 26 | 2.64 | 0.487 | 18.44 | 28 | 2.93 | 0.448 | 15.32 |
| 6.50 | 26 | 2.58 | 0.444 | 17.23 | 28 | 2.94 | 0.532 | 18.07 |
| 7.00 | 26 | 2.48 | 0.444 | 17.88 | 28 | 2.96 | 0.471 | 15.92 |
| 8.00 | 26 | 2.41 | 0.444 | 18.44 | 28 | 2.88 | 0.456 | 15.82 |
| 9.00 | 26 | 2.26 | 0.428 | 18.93 | 28 | 2.83 | 0.523 | 18.47 |
| 10.00 | 26 | 2.22 | 0.409 | 18.45 | 28 | 2.74 | 0.587 | 21.45 |
| 12.00 | 26 | 2.04 | 0.382 | 18.68 | 28 | 2.45 | 0.678 | 27.69 |
| 18.00 | 26 | 1.43 | 0.299 | 20.95 | 28 | 1.44 | 0.373 | 25.90 |
| 24.00 | 26 | 0.998 | 0.243 | 24.39 | 28 | 0.873 | 0.261 | 29.87 |
| 32.00 | 26 | 0.472 | 0.138 | 29.17 | 28 | 0.382 | 0.139 | 36.41 |
| 48.00 | 26 | 0.116 | 0.0442 | 38.18 | 28 | 0.0913 | 0.0557 | 61.00 |

| | | | | | | | | |
|---|---|---|---|---|---|---|---|---|
| Note: Plasma samples analyzed using a bioanalytical method with a validated range 0.0500 to 30.0 ug/mL; concentrations reported in µg/mL to 3 significant figures; concentrations below limit of quantification set to zero (0.00 µg/mL) in the data summarization NC = Not calculated | | | | | | | | |

**Table 11: Pharmacokinetic Parameters of Levetiracetam**

| Parameter | Group 1/TreatmentA: | | | | Group 1/TreatmentB: | | | |
|---|---|---|---|---|---|---|---|---|
| | 190 mg Tablet A, Fasted | | | | 190 mg Tablet A, Fed | | | |
| | n | Mean | SD | CV% | n | Mean | SD | CV% |
| Tₘₐₓ (h) | 28 | 4.39 | 2.05 | 46.71 | 27 | 6.93 | 1.97 | 28.50 |
| Cₘₐₓ (µg/mL) | 28 | 2.31 | 0.505 | 21.83 | 27 | 2.53 | 0.528 | 20.86 |
| Cmax/D (µg/mL/mg) | 28 | 0.0122 | 0.00266 | 21.83 | 27 | 0.0133 | 0.00278 | 20.86 |
| AUCₗₐₛₜ (h^{∗}µg/mL) | 28 | 46.40 | 11.44 | 24.66 | 27 | 46.53 | 9.352 | 20.10 |
| AUClast/D (h^{∗}µg/mL/mg) | 28 | 0.2442 | 0.06024 | 24.66 | 27 | 0.2449 | 0.04922 | 20.10 |
| AUC_{inf} (h^{∗}µg/mL) | 28 | 47.93 | 11.63 | 24.25 | 27 | 47.81 | 9.451 | 19.77 |
| AUCinf/D (h^{∗}µg/mL/mg) | 28 | 0.2523 | 0.06119 | 24.25 | 27 | 0.2516 | 0.04974 | 19.77 |
| AUC_{Extrap} (%) | 28 | 3.29 | 2.52 | 76.75 | 27 | 2.72 | 1.40 | 51.57 |
| λ_{z} (h⁻¹) | 28 | 0.0873 | 0.0114 | 13.11 | 27 | 0.0911 | 0.0096 | 10.50 |
| T_{1/2} (h) | 28 | 8.09 | 1.17 | 14.50 | 27 | 7.70 | 0.88 | 11.39 |
| Tₗₐₛₜ (h) | 28 | 46.82 | 4.19 | 8.94 | 27 | 45.64 | 5.80 | 12.70 |
| Cₗₐₛₜ (µg/mL) | 28 | 0.125 | 0.0661 | 52.74 | 27 | 0.115 | 0.0605 | 52.46 |

| Parameter | Group 2/TreatmentA: | | | | Group 2/Treatment B: | | | |
|---|---|---|---|---|---|---|---|---|
| | 220 mg Tablet D, Fasted | | | | 220 mg Tablet D, Fed | | | |
| | n | Mean | SD | CV% | n | Mean | SD | CV% |
| Tₘₐₓ (h) | 26 | 4.04 | 1.25 | 30.91 | 28 | 6.64 | 1.83 | 27.55 |
| Cₘₐₓ (µg/mL) Cmax/D | 26 | 3.02 | 0.569 | 18.88 | 28 | 3.16 | 0.623 | 19.73 |
| (µg/mL/mg) AUCₗₐₛₜ | 26 | 0.0137 | 0.00259 | 18.88 | 28 | 0.0143 | 0.00283 | 19.73 |
| (h^{∗}µg/mL) AUClast/D | 26 | 56.33 | 10.42 | 18.49 | 28 | 55.27 | 10.35 | 18.72 |
| (h^{∗}µg/mL/mg) AUC_{inf} | 26 | 0.2560 | 0.04734 | 18.49 | 28 | 0.2512 | 0.04702 | 18.72 |
| (h^{∗}µg/mL) AUCinf/D | 26 | 57.68 | 10.67 | 18.50 | 28 | 56.63 | 10.53 | 18.59 |
| (h^{∗}µg/mL/mg) | 26 | 0.2622 | 0.04850 | 18.50 | 28 | 0.2574 | 0.04784 | 18.59 |
| AUC_{Extrap} (%) | 26 | 2.35 | 1.10 | 47.03 | 28 | 2.42 | 1.19 | 48.97 |
| λ_{z} (h⁻¹) | 26 | 0.0905 | 0.0123 | 13.63 | 28 | 0.0933 | 0.0126 | 13.50 |
| T_{1/2} (h) | 26 | 7.81 | 1.14 | 14.59 | 28 | 7.56 | 1.01 | 13.32 |
| Tₗₐₛₜ (h) | 26 | 48.01 | 0.06 | 0.13 | 28 | 45.73 | 5.71 | 12.48 |
| Cₗₐₛₜ (µg/mL) | 26 | 0.116 | 0.0442 | 38.18 | 28 | 0.124 | 0.0640 | 51.46 |

**Table 12: Statistical Analysis of the Natural Log-Transformed Systemic Exposure Parameters of Levetiracetam Comparing Extended-Release Tablet A, 190 mg under Fed Conditions (Treatment B1) to Extended-Release Tablet A, 190 mg under Fasted Conditions (Treatment A1) in Group 1**

| **Dependent Variable** | **Geometric Mean^{a}** | | **Ratio (%)^{b}** | **90% CI^{c}** | | **Power ANOVA** | |
|---|---|---|---|---|---|---|---|
| | **Test** | Ref | **(Test/Ref)** | **Lower** | **Upper** | | **CV%** |
| **ln(Cₘₐₓ)** | 2.4777 | 2.2968 | 107.88 | 103.32 | 112.63 | 1.0000 | 9.29 |
| **ln(AUCₗₐₛₜ)** | 45.6972 | 45.6427 | 100.12 | 95.44 | 105.02 | 1.0000 | 10.31 |
| **ln(AUC_{inf}**) | 46.9703 | 47.0059 | 99.92 | 95.38 | 104.68 | 1.0000 | 10.02 |

| | | | | | | | |
|---|---|---|---|---|---|---|---|
| ^{a} Geometric Mean for 190 mg Tablet A, Fed (Test-B1) and 190 mg Tablet A, Fasted (Ref-A1) based on Least Squares Mean of log-transformed parameter values ^{b} Ratio(%) = Geometric Mean (Test)/Geometric Mean (Ref) ^{c} 90% Confidence Interval | | | | | | | |

**Table 13: Statistical Analysis of the Natural Log-Transformed Systemic Exposure Parameters of Levetiracetam Comparing Extended-Release Tablet D, 220 mg under Fed Conditions (Treatment B2) to Extended-Release Tablet D, 220 mg under Fasted Conditions (Treatment A2) in Group 2**

| **Dependent Variable** | **Geometric Mean^{a}** | | **Ratio (%)^{b}** | **90% CI^{c}** | | **Power ANOVA** | |
|---|---|---|---|---|---|---|---|
| | **Test** | Ref | **(Test/Ref)** | **Lower** | **Upper** | | **CV%** |
| **ln(Cₘₐₓ)** | 3.1117 | 2.9660 | 104.91 | 100.32 | 109.72 | 1.0000 | 9.43 |
| **ln(AUCₗₐₛₜ)** | 54.5598 | 55.6286 | 98.08 | 94.03 | 102.30 | 1.0000 | 8.86 |
| **ln(AUC_{inf})** | 55.9406 | 56.9747 | 98.18 | 94.21 | 102.33 | 1.0000 | 8.71 |

| | | | | | | | |
|---|---|---|---|---|---|---|---|
| ^{a} Geometric Mean for 220 mg Tablet D, Fed (Test-B2) and 220 mg Tablet D, Fasted (Ref-A2) based on Least Squares Mean of log-transformed parameter values ^{b} Ratio(%) = Geometric Mean (Test)/Geometric Mean (Ref) ^{c} 90% Confidence Interval | | | | | | | |

**Table 14: Statistical Analysis of the Natural Log-Transformed Systemic Exposure Dose-Normalized Parameters of Levetiracetam Comparing Extended-Release Tablet D, 220 mg under Fasted Conditions (Treatment A2) to Extended-Release Tablet A, 190 mg under Fasted Conditions (Treatment A1)**

| **Dependent Variable** | **Geometric Mean^{a}** | | **Ratio (%)^{b}** | **90% CI^{c}** | | **Power ANOVA** | |
|---|---|---|---|---|---|---|---|
| | **Test** | **Ref** | **(Test/Ref)** | **Lower** | **Upper** | | **CV%** |
| **ln(Cₘₐₓ/D)** | 0.0135 | 0.0119 | 113.39 | 102.88 | 124.98 | 0.9825 | 21.58 |
| **ln(AUCₗₐₛₜ/D)** | 0.2518 | 0.2371 | 106.22 | 95.98 | 117.54 | 0.9754 | 22.49 |
| **ln(AUC_{inf}/D)** | 0.2579 | 0.2452 | 105.17 | 95.21 | 116.16 | 0.9789 | 22.07 |

| | | | | | | | |
|---|---|---|---|---|---|---|---|
| ^{a} Geometric Mean for 190 mg Tablet A, Fasted (Test-A2) and 220 mg Tablet D, Fasted (Ref-A1) based on Least Squares Mean of log-transformed parameter values ^{b} Ratio(%) = Geometric Mean (Test)/Geometric Mean (Ref) ^{c} 90% Confidence Interval | | | | | | | |

**Table 15: Statistical Analysis of the Natural Log-Transformed Systemic Exposure Dose-Normalized Parameters of Levetiracetam Comparing Extended-Release Tablet D, 220 mg under Fed Conditions (Treatment B2) to Extended-Release Tablet D, 190 mg under Fed Conditions (Treatment B1)**

| **Dependent Variable** | **Geometric Mean^{a}** | | **Ratio (%)^{b}** | **90% CI^{c}** | | **Power ANOVA** | |
|---|---|---|---|---|---|---|---|
| | **Test** | Ref | **(Test/Ref)** | **Lower** | **Upper** | | **CV%** |
| **ln(Cₘₐₓ/D)** | 0.0141 | 0.0130 | 108.14 | 98.72 | 118.47 | 0.9906 | 20.41 |
| **ln(AUCₗₐₛₜ/D)** | 0.2472 | 0.2404 | 102.82 | 94.49 | 111.89 | 0.9959 | 18.87 |
| **ln(AUC_{inf}/D)** | 0.2534 | 0.2472 | 102.51 | 94.32 | 111.42 | 0.9965 | 18.61 |

| | | | | | | | |
|---|---|---|---|---|---|---|---|
| ^{a} Geometric Mean for 220 mg Tablet D, Fasted (Test-B2) and 220 mg Tablet D, Fasted (Ref-B1) based on Least Squares Mean of log-transformed parameter values ^{b} Ratio(%) = Geometric Mean (Test)/Geometric Mean (Ref) ^{c} 90% Confidence Interval | | | | | | | |

Preferred embodiments of the present invention are described below and are referred to as embodiments E1 to E28:
E1. An extended release pharmaceutical composition comprising:
   a) 220 mg of levetiracetam;
   b) 280 mg-350 mg of hydroxypropyl methylcellulose;
   c) 1.2 mg-1.4 mg of colloidal silicon dioxide;
   d) 92.8 mg-119.2 mg of silicified microcrystalline cellulose; and
   e) 6.0 mg-6.7 mg of magnesium stearate.
E2. An extended release pharmaceutical composition comprising:
   a) 220 mg of levetiracetam;
   b) 280 mg of hydroxypropyl methylcellulose;
   c) 1.2 mg of colloidal silicon dioxide;
   d) 92.8 mg of silicified microcrystalline cellulose; and
   e) 6.0 mg of magnesium stearate.
E3. An extended release pharmaceutical composition comprising:
   a) 220 mg of levetiracetam;
   b) 347.5 mg of hydroxypropyl methylcellulose;
   c) 1.4 mg of colloidal silicon dioxide;
   d) 119.2 mg of silicified microcrystalline cellulose; and
   e) 6.7 mg of magnesium stearate.
E4. The pharmaceutical composition of any one of E1-3, wherein the hydroxypropyl methylcellulose is Methocel™ K15M CR or Methocel™ K100M Premium CR.
E5. The pharmaceutical composition of any one of E1-3, wherein the hydroxypropyl methylcellulose is Methocel™ K15M CR.
E6. The pharmaceutical composition of any one of E1-5, wherein the silicified microcrystalline cellulose is ProSolv™ HD90.
E7. An extended release pharmaceutical composition comprising:
   a) 190 mg of levetiracetam;
   b) 300 mg of hydroxypropyl methylcellulose;
   c) 1.2 mg of colloidal silicon dioxide;
   d) 102.8 mg of silicified microcrystalline cellulose; and
   e) 6 mg of magnesium stearate.
E8. An extended release pharmaceutical composition comprising:
   a) 190 mg of levetiracetam;
   b) 300 mg of hydroxypropyl methylcellulose;
   c) 1.2 mg of colloidal silicon dioxide;
   d) 102.8 mg of anhydrous dicalcium phosphate; and
   e) 6 mg of magnesium stearate.
E9. The pharmaceutical composition of E7 or E8, wherein the hydroxypropyl methylcellulose is Methocel™ K15M CR or Methocel™ K100M Premium CR.
E10. The pharmaceutical composition of E7 or E8, wherein the hydroxypropyl methylcellulose is Methocel™ K15M CR.
E11. The pharmaceutical composition of any one of E7, E9, and E10, wherein the silicified microcrystalline cellulose is ProSolv™ HD90.
E12. The pharmaceutical composition of any one of E1-11, wherein the composition is formulated for once daily administration.
E13. The pharmaceutical composition of any one of E1-11, wherein the composition is formulated for one-unit-dosage-form-once-daily administration.
E14. The pharmaceutical composition of any one of E1-13, wherein the composition is in the form of a tablet.
E15. The pharmaceutical composition of E14, wherein the composition is formulated for one-tablet-once-daily administration.
E16. The pharmaceutical composition of any one of E1-15, wherein the composition is formulated for oral administration.
E17. The pharmaceutical composition of any one of E1-16, wherein the composition does not comprise a hydrophobic rate controlling polymer.
E18. The pharmaceutical composition of any one of E1-16, wherein the composition does not comprise a functional coating.
E19. A method of improving cognition in a subject suffering from cognitive impairment or at risk thereof, wherein the method comprises administering the pharmaceutical composition of any one of E1-18.
E20. The method of E19, wherein the subject suffers from cognitive impairment associated with a central nervous system (CNS) disorder, or at risk thereof.
E21. The method of E19 or E20, wherein the cognitive impairment is associated with age-related cognitive impairment.
E22. The method of E21, wherein the age-related cognitive impairment is Mild Cognitive Impairment.
E23. The method of E22, wherein the Mild Cognitive Impairment is amnestic Mild Cognitive Impairment.
E24. The method of E19 or E20, wherein the cognitive impairment is associated with dementia, Alzheimer's disease, schizophrenia, amyotrophic lateral sclerosis, post traumatic stress disorder, cancer therapy, bipolar disorder mental retardation, Parkinson's disease, autism, compulsive behavior, or substance addiction.
E25. A method of treating mild cognitive impairment due to Alzheimer's disease in a human subject in need thereof, wherein the method comprises administering the pharmaceutical composition of any one of E1-18.
E26. A method of treating amnestic mild cognitive impairment due to Alzheimer's disease in a human subject in need thereof, wherein the method comprises administering the pharmaceutical composition of any one of E1-18.
E27. A method of slowing the progression of mild cognitive impairment due to Alzheimer's disease in a human subject in need thereof, wherein the method comprises administering the pharmaceutical composition of any one of E1-18.
E28. A method of slowing the progression of amnestic mild cognitive impairment due to Alzheimer's disease in a human subject in need thereof, wherein the method comprises administering the pharmaceutical composition of any one of E1-18.

## Claims

1. An extended release pharmaceutical composition comprising levetiracetam, wherein the composition at steady state provides a plasma concentration of levetiracetam in a subject of between 1.9 µg/ml and 4.4 µg/ml for at least 12 consecutive hours of the 14 hours after administration, and wherein the composition is formulated for once daily administration.

2. An extended release pharmaceutical composition comprising levetiracetam, wherein the composition at steady state provides a plasma concentration of levetiracetam in a subject of between 1.9 µg/ml and 4.4 µg/ml for at least 14 consecutive hours of the 16 hours after administration, and wherein the composition is formulated for once daily administration.

3. The pharmaceutical composition of claim 1 or 2 which is formulated for oral administration.

4. The pharmaceutical composition of any one of claims 1-3 for use in a method of improving cognition in a subject suffering from cognitive impairment or at risk thereof.

5. The pharmaceutical composition for use according to claim 4, wherein the cognitive impairment is associated with a central nervous system (CNS) disorder.

6. The pharmaceutical composition for use according to claim 4, wherein the cognitive impairment is associated with age-related cognitive impairment.

7. The pharmaceutical composition for use according to claim 6, wherein the age-related cognitive impairment is Mild Cognitive Impairment.

8. The pharmaceutical composition for use according to claim 7, wherein the Mild Cognitive Impairment is amnestic Mild Cognitive Impairment.

9. The pharmaceutical composition for use according to claim 4 or 5, wherein the cognitive impairment is associated with dementia, Alzheimer's disease, schizophrenia, amyotrophic lateral sclerosis, post traumatic stress disorder, cancer therapy, bipolar disorder, mental retardation, Parkinson's disease, autism, compulsive behavior, or substance addiction.

10. The pharmaceutical composition of any one of claims 1-3 for use in a method of treating mild cognitive impairment in a human subject in need thereof.

11. The pharmaceutical composition for use according to claim 10, wherein the mild cognitive impairment is amnestic mild cognitive impairment.

12. The pharmaceutical composition of any one of claims 1-3 for use in a method of slowing the progression of mild cognitive impairment in a human subject in need thereof.

13. The pharmaceutical composition for use according to claim 12, wherein the mild cognitive impairment is amnestic mild cognitive impairment.
